# EUROPEAN PATENT APPLICATION

(11) **EP 2 767 832 A1**
(43) Date of publication of application: **20.08.2014**
(21) Application number: 13305183.9
(22) Date of filing: 18.02.2013
(51) Int. Cl.: G01N 33/68, H01J 49/00

(54) **Photo or chemolabile conjugates for molecules detection**

(71) Applicant: Imabiotech, 59120 Loos (FR); Université des Sciences et Technologies De Lille - Lille 1, 59655 Villeneuve d'Ascq (FR); Centre National de la Recherche Scientifique, 75016 Paris (FR)
(72) Inventor: Stauber, Jonathan, 59800 Lille (FR); Ebran, Jean-Philippe, 59000 Lille (FR); Melnyk, Oleg, 59112 Annoeullin (FR)
(74) Representative: Tezier Herman, Béatrice

(57) **Abstract**

The present invention relates to the field of the detection of molecules of interest in a sample, preferably by mass spectrometry. The present invention concerns a label compound, a molecule labeled with said compound (a conjugate), a method of detection of a molecule of interest (a target molecule) in a sample involving said conjugate, a kit to implement said method and a process for the preparation of the label.

SEQUENCE LISTING

<110>  IMABIOTECH

<120>  Photo or chemolabile conjugates for molecules detection

<130>  B1555

<160>  11

<170>  PatentIn version 3.5

<210>  1
<211>  8
<212>  PRT
<213>  artificial sequence

<220>
<223>  SEQ ID NO: 1


<220>
<221>  MISC_FEATURE
<222>  (3)..(3)
<223>  Xaa may be an amino acid selected from the group consisting of
       Alanine (Ala, A), serine (Ser, S), valine (Val, V), asparagine
       (Asn, N), glutamine (Gln, Q), leucine (Leu, L) and proline (Pro,
       P)

<220>
<221>  MISC_FEATURE
<222>  (7)..(7)
<223>  Xaa may be an amino acid selected from the group consisting of
       Alanine (Ala, A), serine (Ser, S), valine (Val, V), asparagine
       (Asn, N), glutamine (Gln, Q), leucine (Leu, L) and proline (Pro,
       P)

<400>  1

Gly Arg Xaa Phe Arg Gly Xaa Gly
1               5


<210>  2
<211>  8
<212>  PRT
<213>  artificial sequence

<220>
<223>  SEQ ID NO: 2

<400>  2

Gly Arg Ala Phe Arg Gly Ala Gly
1               5


<210>  3
<211>  8
<212>  PRT
<213>  artificial sequence

<220>

<223>  SEQ ID NO: 3

<400>  3

Gly Arg Ala Phe Arg Gly Ser Gly
1               5


<210>  4
<211>  8
<212>  PRT
<213>  artificial sequence

<220>
<223>  SEQ ID NO: 4

<400>  4

Gly Arg Ala Phe Arg Gly Val Gly
1               5


<210>  5
<211>  8
<212>  PRT
<213>  artificial sequence

<220>
<223>  SEQ ID NO: 5

<400>  5

Gly Arg Ala Phe Arg Gly Asn Gly
1               5


<210>  6
<211>  8
<212>  PRT
<213>  artificial sequence

<220>
<223>  SEQ ID NO: 6

<400>  6

Gly Arg Ala Phe Arg Gly Gln Gly
1               5


<210>  7
<211>  8
<212>  PRT
<213>  artificial sequence

<220>
<223>  SEQ ID NO: 7

<400>  7

Gly Arg Pro Phe Arg Gly Leu Gly
1               5

<210>  8
<211>  8
<212>  PRT
<213>  artificial sequence

<220>
<223>  SEQ ID NO: 8

<400>  8

Gly Arg Asn Phe Arg Gly Asn Gly
1               5


<210>  9
<211>  8
<212>  PRT
<213>  artificial sequence

<220>
<223>  SEQ ID NO: 9

<400>  9

Gly Arg Gln Phe Arg Gly Asn Gly
1               5


<210>  10
<211>  8
<212>  PRT
<213>  artificial sequence

<220>
<223>  SEQ ID NO: 10

<400>  10

Gly Arg Gln Phe Arg Gly Gln Gly
1               5


<210>  11
<211>  8
<212>  PRT
<213>  artificial sequence

<220>
<223>  SEQ ID NO: 11

<400>  11

Gly Arg Gln Phe Arg Gly Phe Gly
1               5

## Description

### Introduction

The present invention relates to the field of the detection of molecules of interest in a sample, preferably by mass spectrometry. The present invention concerns a label compound, a molecule labeled with said compound (a conjugate), a method of detection of a molecule of interest (a target molecule) in a sample involving said conjugate, a kit to implement said method and a process for the preparation of the label.

### Background of the invention

The detection of molecules in a sample, for instance in a biological sample, is of great interest, in particular in the analysis and medical fields.
Different methods have already been developed to detect the presence of molecules in a sample. Some methods include marking the molecule with a tag and detecting the release of said tag with appropriate techniques. For instance, release of the tag may be detected by colorimetry, by fluorimetry and/or by mass spectrometry. The release of the tag can be triggered by different activation types or stimuli, such as biochemical processes, chemical activation or irradiation at a specific wavelength.

In that context, different strategies have been developed to allow the coupling of:
- A moiety that is able to link, in particular to specifically link, a molecule of interest,
- A self-immolative trigger moiety that can be activated by at least one stimulus, for instance it can be cleaved by or reacts to said at least one stimulus, and
- A tag moiety that is released in the medium when the trigger moiety is subjected to the appropriate stimulus.

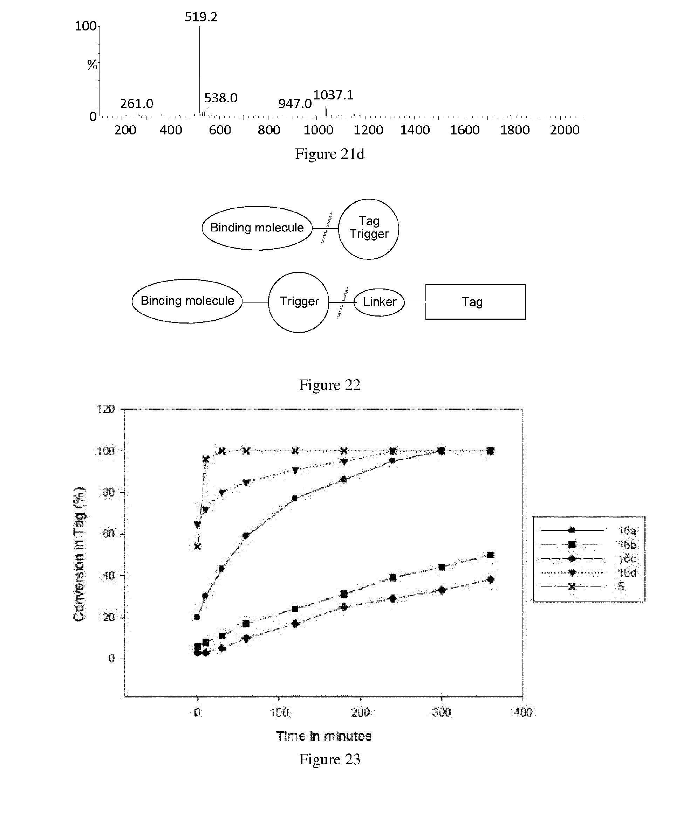

Two main strategies are to be noticed, which are presented on Figure 22.
In the first strategy, the tag and trigger moieties are the same. The stimulus simultaneously activates the trigger and releases the tag.
In the second strategy, the trigger moiety is situated between the tag moiety and the moiety able to link a molecule of interest. Activation of the trigger with the stimulus leads to fragmentation of the compound at the level of the trigger moiety, and subsequent release of the tag moiety.

The above presented strategies afford the possibility to release and detect a tag after a stimulus is applied, in order to detect a molecule linked to the compound. The chemical structure of the compound, in particular of the trigger moiety, depends on the type of stimulus used to activate it. Consequently, one trigger is appropriate for one specific type of stimulus. Trigger moiety modification in order to allow the use of a different stimulus for the tag release requires important chemical modifications, leading to the change of the compound as a whole (i.e. not only the trigger but the linker and the tag).

The Applicants have now developed a novel strategy for coupling the three moieties of the compound (moiety able to link a molecule of interest, trigger and tag). In this strategy, the trigger moiety is inserted in a lateral chain of the compound. Said strategy affords the possibility to easily modify the nature and/or structure of the trigger moiety independently of the rest of the compound, in particular of the tag. The resulting compound can thus be easily adapted to different stimuli.

### Brief description of the figures

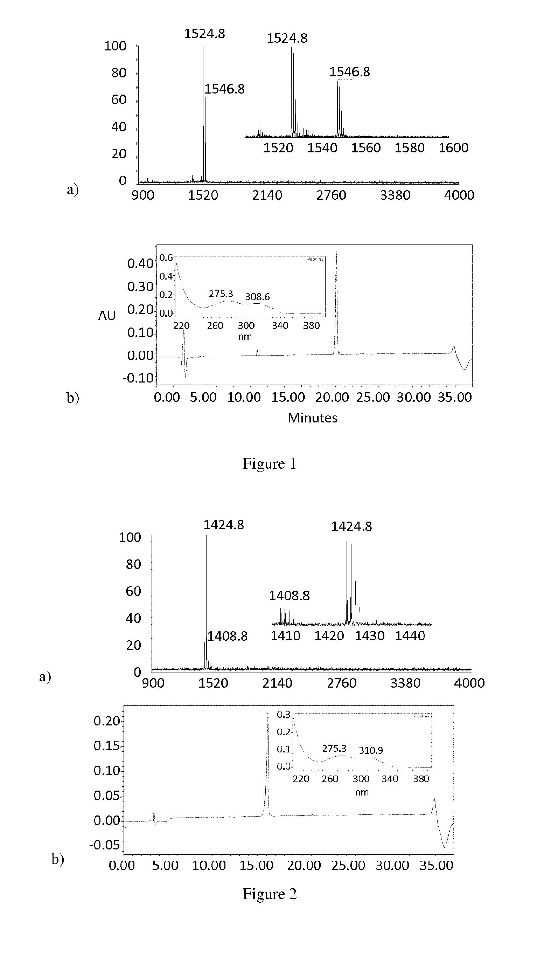

Figure 1: Characterization of compound 5. a) MALDI-TOF spectrum - MALDI-TOF matrix: CHCA - Calculated for C₇₀H₉₇N₁₉O₂₀ [M+H]⁺ 1524.7, found 1524.8 ; [M+Na]⁺ calculated for 1546.7, found 1546.8. b) RP-HPLC Analysis, RT=20.9 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm).
Figure 2: Characterization of compound 6. a) MALDI-TOF spectrum MALDI-TOF matrix: CHCA - Calculated for C₆₅H₈₉N₁₉O₁₈ [M+H]⁺ 1424.7, found 1424.8 ; [M+H-O]⁺ calculated 1408.7, found 1408.8. b) RP-HPLC Analysis, RT=16.1 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm).

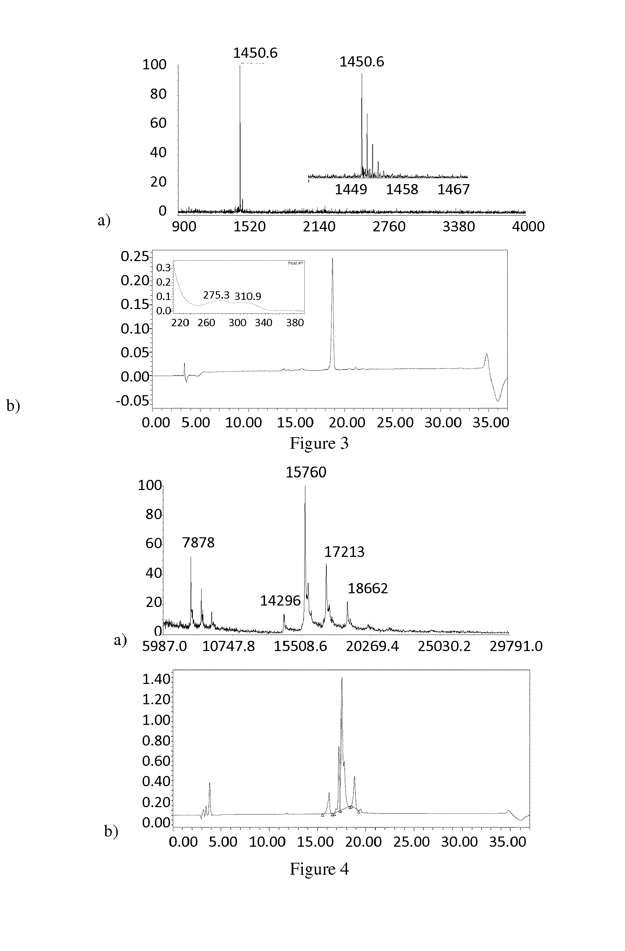

Figure 3: Characterization of compound 7. a) MALDI-TOF spectrum - MALDI-TOF matrix: CHCA - Calculated for C₇₀H₉₂N₂₀O₂₂ [M+H-NHS]⁺ 1450.7, found 1450.6. b) RP-HPLC Analysis RT=18.8 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm).
Figure 4: Characterization of compound 8. a) MALDI-TOF spectrum - MALDI-TOF matrix: 3,5-dimethoxy-4-hydroxycinnamic acid. b) RP-HPLC Analysis, labeled lysozyme RT=16.9 min. Buffer A H₂O-0.05% TFA / Buffer B 0.05% TFA CH₃CN/water (4/1) column C18 Nucleosil 100 Å 5 µm (2.1x250 mm) with a linear gradient 0-100% B in 30 minutes (1.0 mL/min, detection from 210 to 400 nm).

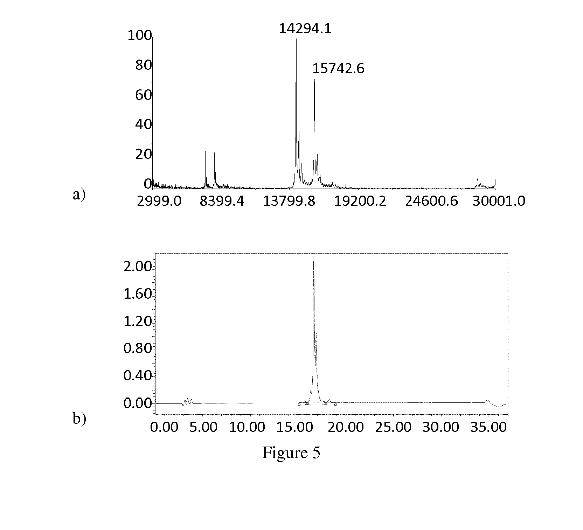

Figure 5: Characterization of compound **8a.** a) MALDI-TOF spectrum - MALDI-TOF matrix: 3,5-dimethoxy-4-hydroxycinnamic acid. b) RP-HPLC Analysis, Lysozyme RT=16.6 min., labeled lysozyme RT=16.9 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm).

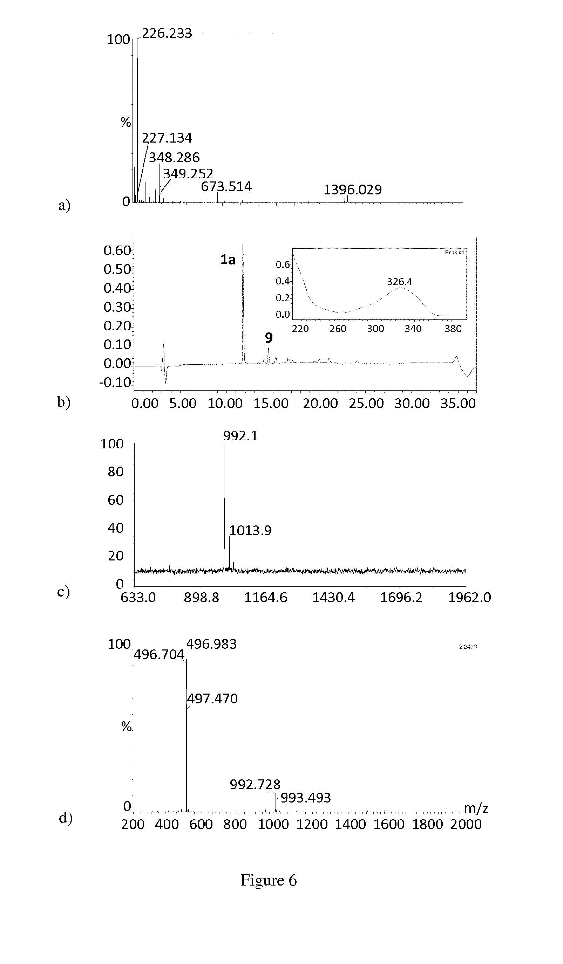

Figure 6: Characterization of compounds **1a** and **9**. a) ESI Analysis of product 9 (RT= 14.4 min.) - Calculated for C₁₆H₂₇N₃O₄ [M+H-Boc]⁺ 226.2, found 226.2. [M+Na]⁺ calculated 348.2, found 348.3. b) RP-HPLC Analysis, **1a** RT=11.7 min., 9 RT=14.4 min. Buffer A H₂O-0.05% TFA / Buffer B 0.05% TFA CH₃CN/water (4/1) column C18 Nucleosil 100 Å 5 µm (2.1x250 mm) with a linear gradient 0-100% B in 30 minutes (1.0 mL/min, detection from 210 to 400 nm). c) MALDI-TOF of tag peptide **1a** matrix: CHCA - Calculated for C₄₄H₆₁N₁₅O₁₂ [M+H]⁺ 992.5, found 992.1. [M+Na]⁺ calculated 1014.4, found 1013.9. d) ESI Analysis of product **1a** (RT= 11.7 min.) Calculated for C₄₄H₆₁N₁₅O₁₂ [M+H]⁺ 992.5, found 992.7.

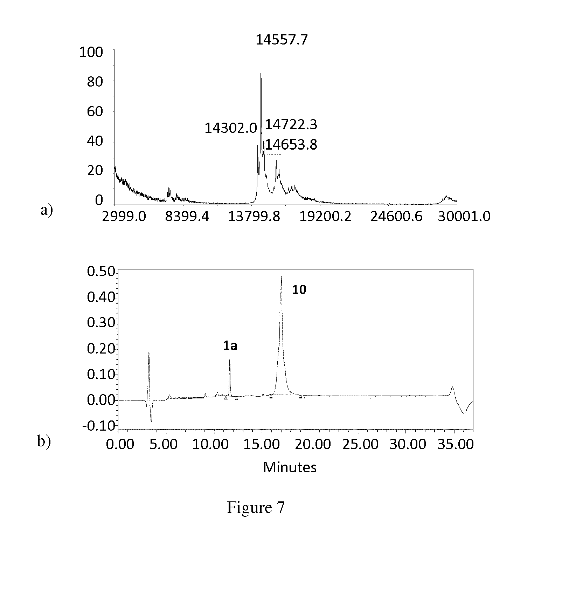

Figure 7: Characterization of compounds **1a** and **10.** a) MALDI-TOF of tag peptide **1a** - MALDI-TOF matrix: 3,5-dimethoxy-4-hydroxycinnamic acid. b) RP-HPLC Analysis, **1a** RT=11.7 min. **10** RT=17.1 min. Buffer A H₂O-0.05% TFA / Buffer B 0.05% TFA CH₃CN/water (4/1) on column C18 Nucleosil 100 Å 5 µm (2.1x250 mm) with a linear gradient 0-100% B in 30 minutes (1.0 mL/min, detection from 210 to 400 nm).

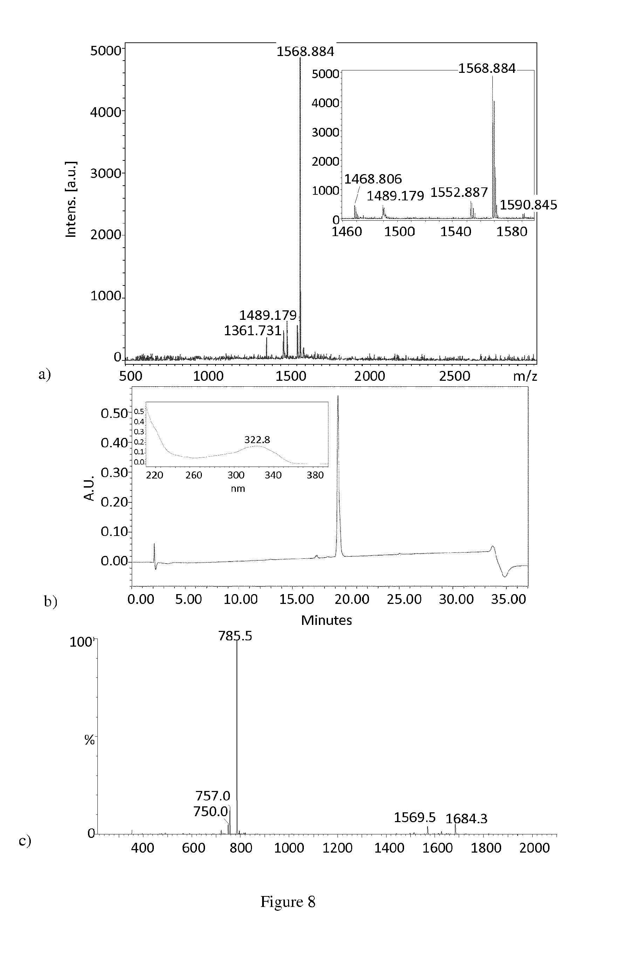

Figure 8: Characterization of compound **16a**. a) MALDI-TOF spectrum - MALDI-TOF matrix: CHCA - Calculated for C₇₂H₁₀₁N₁₉O₂₁ [M+H]⁺ 1568.7, found 1568.9. b) RP-HPLC Analysis, RT=19.2 min. Buffer A H₂O-0.05% TFA / Buffer B 0.05% TFA CH₃CN/water (4/1) column C18 Nucleosil 100 Å 5 µm (2.1x250 mm) with a linear gradient 0-100% B in 30 minutes (1.0 mL/min, detection from 210 to 400 nm). c) ESI spectrum calculated for [M+2H]⁺/2 784.8, found 785.5, [M+H]⁺ calculated 1568.7, found 1569.5.

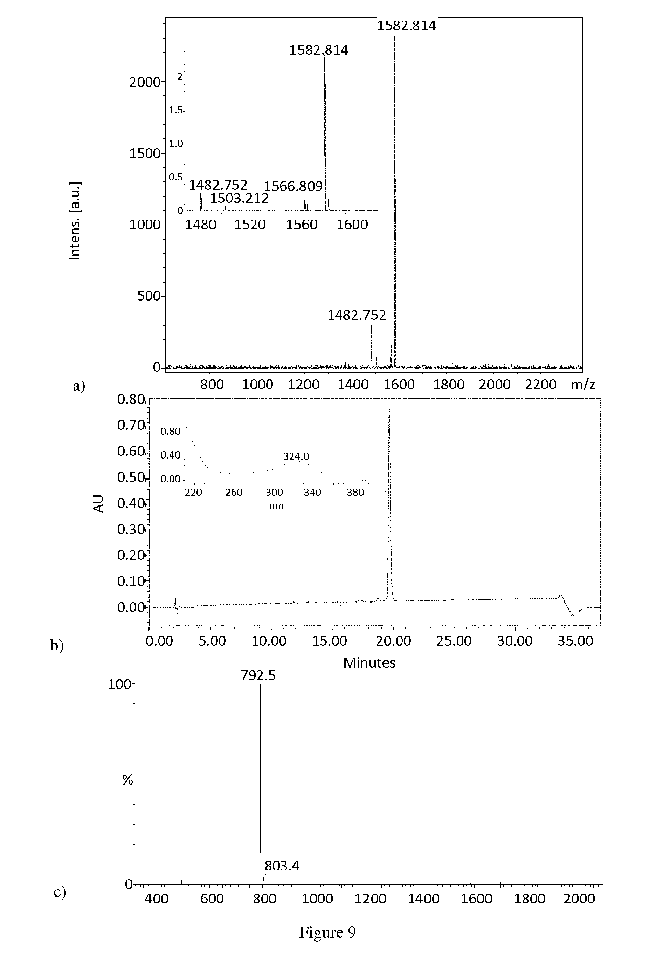

Figure 9: Characterization of compound 16b. a) MALDI-TOF spectrum - MALDI-TOF matrix: CHCA - Calculated for C₇₃H₁₀₃N₁₉O₂₁ [M+H]⁺ 1582.8, found 1582.8. b) RP-HPLC Analysis, RT=19.6 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm). c) ESI spectrum calculated for [M+2H]⁺/2 791.9, found 792.5.

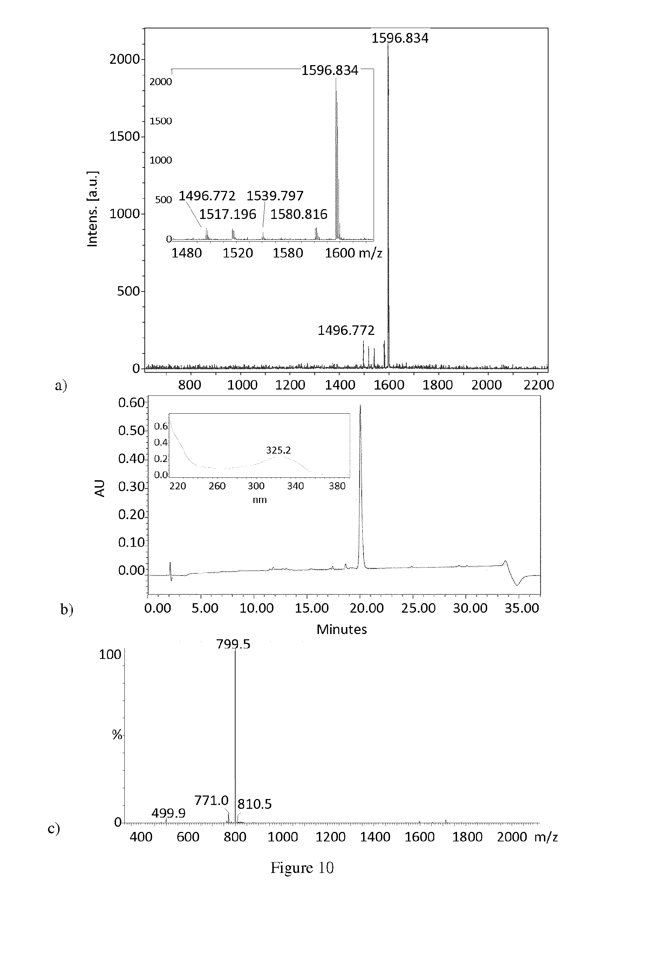

Figure 10: Characterization of compound **16c.** a) MALDI-TOF spectrum - MALDI-TOF matrix: CHCA - Calculated for C₇₄H₁₀₅N₁₉O₂₁ [M+H]⁺ 1596.8, found 1596.8. b) RP-HPLC Analysis, RT=20.0 min. Buffer A H₂O-0.05% TFA / Buffer B 0.05% TFA CH₃CN/water (4/1) column C18 Nucleosil 100 Å 5 µm (2.1x250 mm) with a linear gradient 0-100% B in 30 minutes (1.0 mL/min, detection from 210 to 400 nm). c) ESI spectrum calculated for [M+2H]⁺/2 798.9, found 799.5.

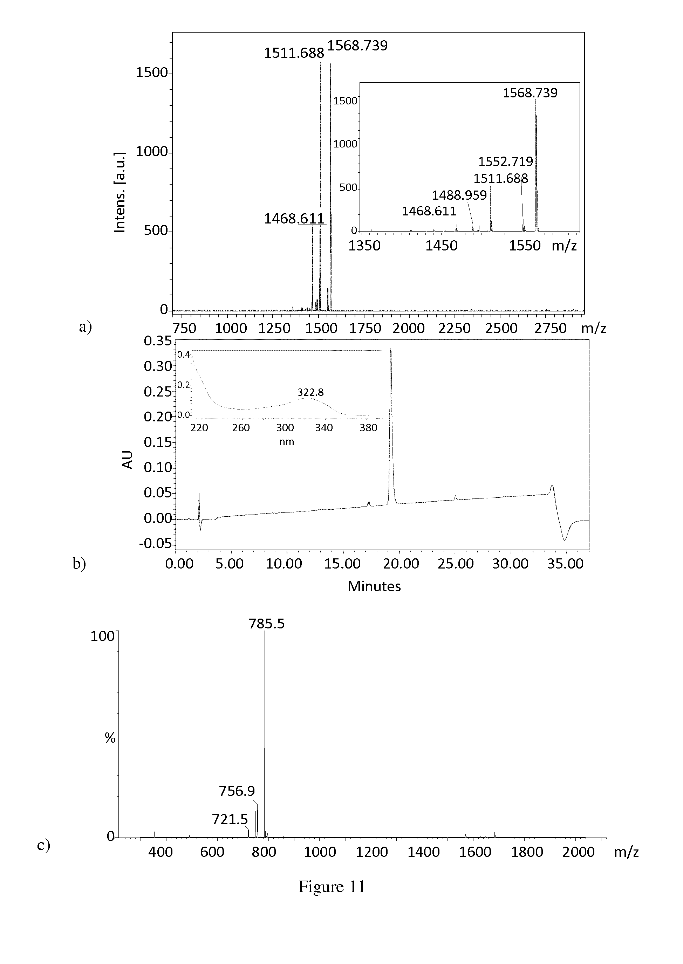

Figure 11: Characterization of compound **16d.** a) MALDI-TOF spectrum - MALDI-TOF matrix: CHCA - Calculated for C₇₂H₁₀₁N₁₉O₂₁ [M+H]⁺ 1568.7, found 1568.7. b) RP-HPLC Analysis, RT=19.2 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm). c) ESI spectrum calculated for [M+2H]⁺/2 784.8, found 785.5.

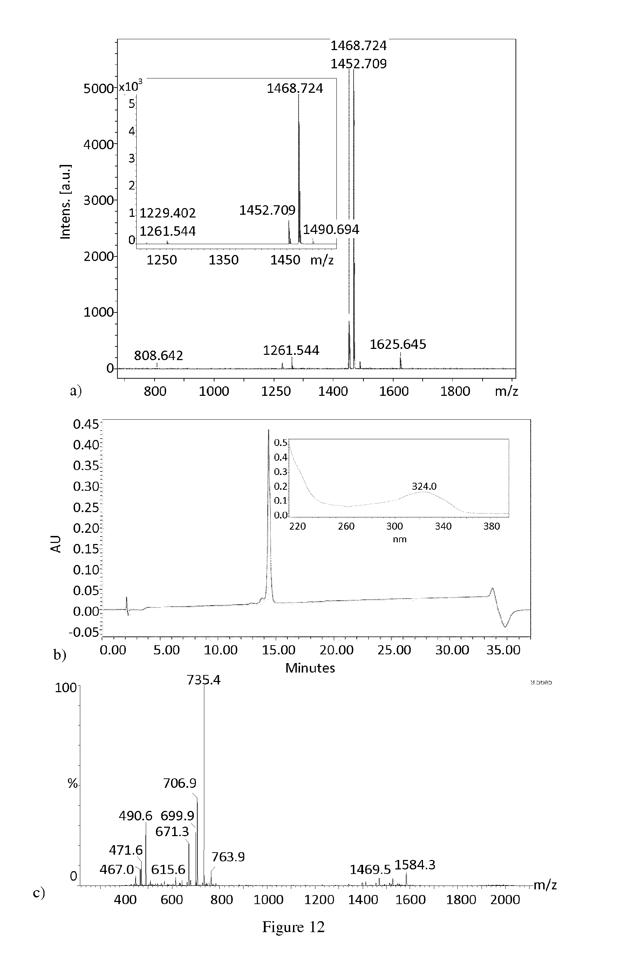

Figure 12: Characterization of compound 17. a) MALDI-TOF spectrum - MALDI-TOF matrix: CHCA - Calculated for C₆₇H₉₃N₁₉O₁₉ [M+H]⁺ 1468.7, found 1468.7. b) RP-HPLC Analysis, RT=14.4 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm). c) ESI spectrum calculated for [M+2H]⁺/2 734.8, found 735.4.

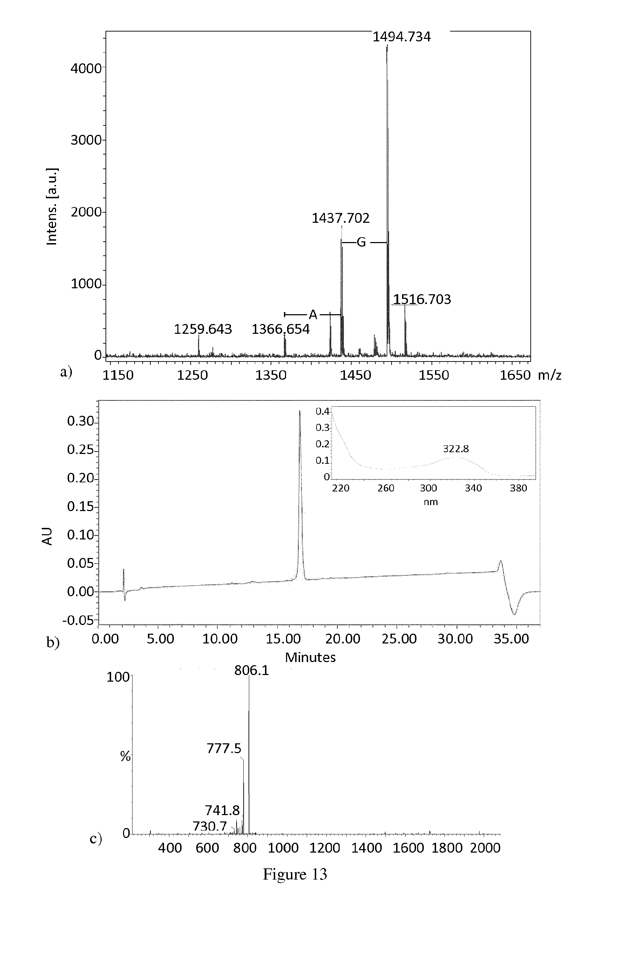

Figure 13: Characterization of compound 18. a) MALDI-TOF spectrum - MALDI-TOF matrix: CHCA - Calculated for C₆₈H₉₁N₁₉O₂₀ [M+H-NHS]⁺ 1494.7, found 1494.7. b) RP-HPLC Analysis, RT=16.9 min. Buffer A H₂O-0.05% TFA / Buffer B 0.05% TFA CH₃CN/water (4/1) column C18 Nucleosil 100 Å 5 µm (2.1x250 mm) with a linear gradient 0-100% B in 30 minutes (1.0 mL/min, detection from 210 to 400 nm). c) ESI spectrum calculated for C₇₂H₉₆N₂₀O₂₃ [M+2H]⁺/2 805.3, found 806.1.

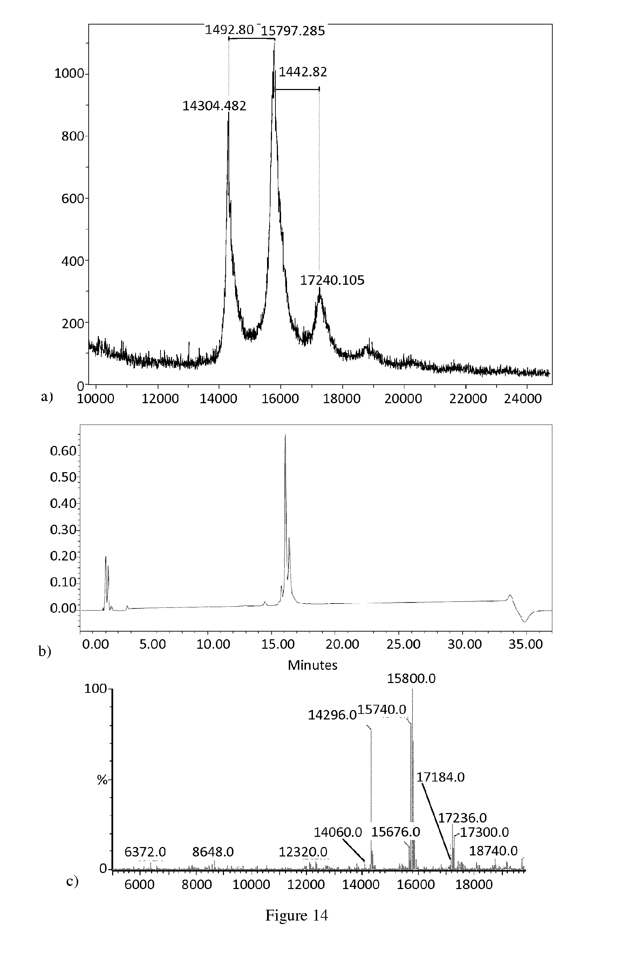

Figure 14: Characterization of compound **19.** a) MALDI-TOF spectrum - MALDI-TOF matrix: 3,5-dimethoxy-4-hydrohycinnamic acid. b) RP-HPLC Analysis, Lysozyme RT=16.6 min., labeled lysozyme 17.1 min. Buffer A H₂O-0.05% TFA / Buffer B 0.05% TFA CH₃CN/water (4/1) column C18 Nucleosil 100 Å 5 µm (2.1x250 mm) with a linear gradient 0-100% B in 30 minutes (1.0 mL/min, detection from 210 to 400 nm). c) ESI spectrum of **19.**

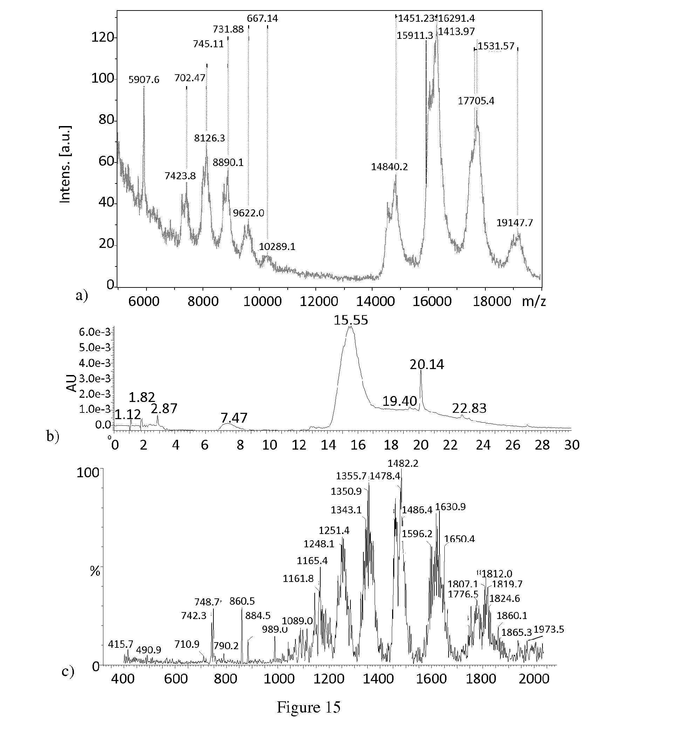

Figure 15: Characterization of compound **20.** a) MALDI-TOF spectrum - MALDI-TOF matrix: 3,5-dimethoxy-4-hydrohycinnamic acid. b) RP-HPLC Analysis, labeled NeutrAvidin 15.6 min. Buffer A H₂O-0.05% Formic Acid / Buffer B 0.05% Formic Acid CH₃CN/water (4/1) column C3 Zorbax 300 SB, 3.5 µm (4.6×150 mm), 50°C, with a linear gradient 0-100% B in 30 minutes (1.0 mL/min, detection from 210 to 400 nm). c) ESI spectrum of **20.**

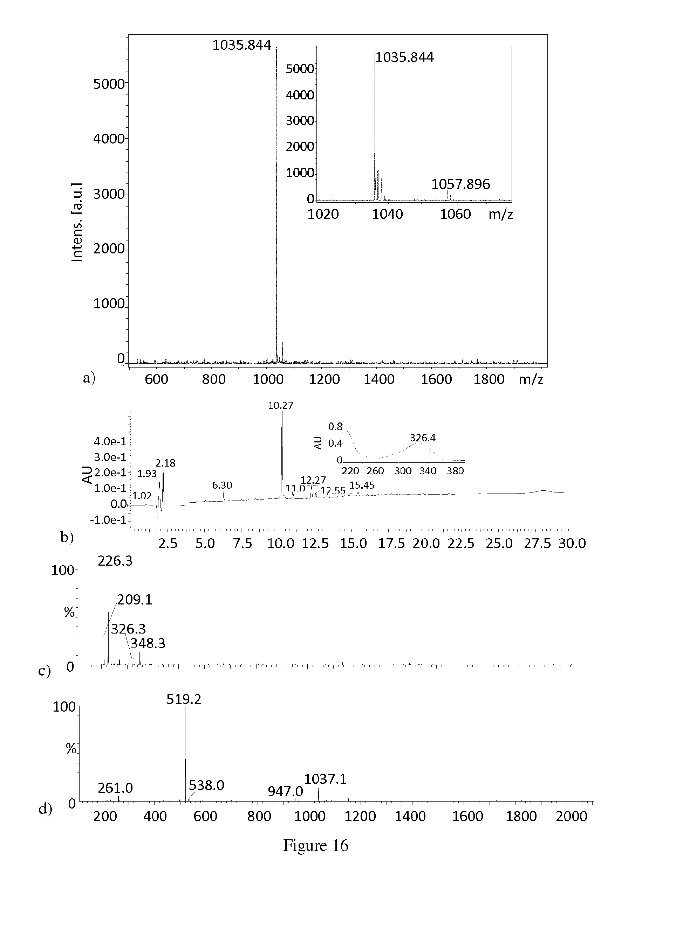

Figure 16: Characterization of compounds **9a** and **21a**. a) MALDI-TOF spectrum of **21a** - MALDI-TOF matrix: CHCA - Calculated for C₄₆H₆₅N₁₅O₁₃ [M+H]⁺ 1036.5, found 1035.8. b) RP-HPLC Analysis, **21a** RT=10.3 min. and **9a** RT= 12.3 min. Buffer A H₂O-0.05% TFA / Buffer B 0.05% TFA CH₃CN/water (4/1) column C18 Nucleosil 100 Å 5 µm (2.1x250 mm) with a linear gradient 0-100% B in 30 minutes (1.0 mL/min, detection from 210 to 400 nm). c) ESI spectrum of **9a** calculated for C₁₆H₂₇N₃O₄ [M+Na]⁺ 348.2, found 348.3 ; [M+H-Boc]⁺ calculated for 226.1, found 226.3. d) ESI spectrum of **21a** calculated for C₄₆H₆₅N₁₅O₁₃ [M+H]⁺ 1036.5, found 1037.1 ; calculated for [M+2H]⁺/2 518.7, found 519.2.

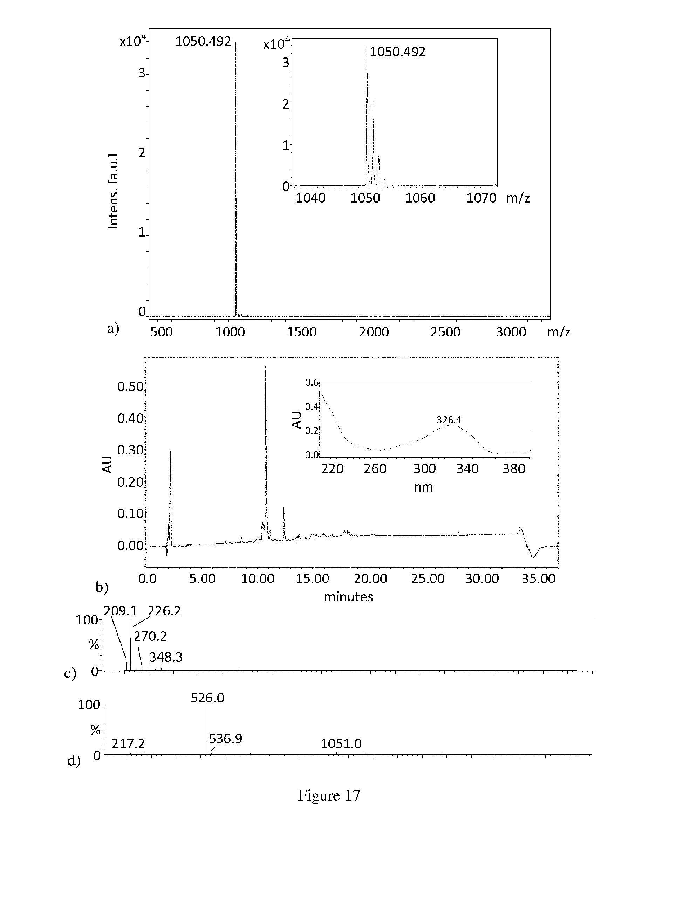

Figure 17: Characterization of compounds **9a** and 21b. a) MALDI-TOF spectrum of **21b -** MALDI-TOF matrix: CHCA - Calculated for C₄₇H₆₇N₁₅O₁₃ [M+H]⁺ 1050.5, found 1050.5. b) RP-HPLC Analysis, **21b** RT=11.0 min. and **9a** RT= 12.3 min. Buffer A H₂O-0.05% TFA / Buffer B 0.05% TFA CH₃CN/water (4/1) column C18 Nucleosil 100 Å 5 µm (2.1x250 mm) with a linear gradient 0-100% B in 30 minutes (1.0 mL/min, detection from 210 to 400 nm). c) ESI spectrum of **9a** calculated for C₁₆H₂₇N₃O₄ [M+Na]⁺ 348.2, found 348.3 ; [M+H-Boc]⁺ calculated for 226.1, found 226.2. d) ESI spectrum of **21b** calculated for C₄₇H₆₇N₁₅O₁₃ [M+H]⁺ 1050.5, found 1051.0 ; calculated for [M+2H]⁺/2 525.7, found 526.0.

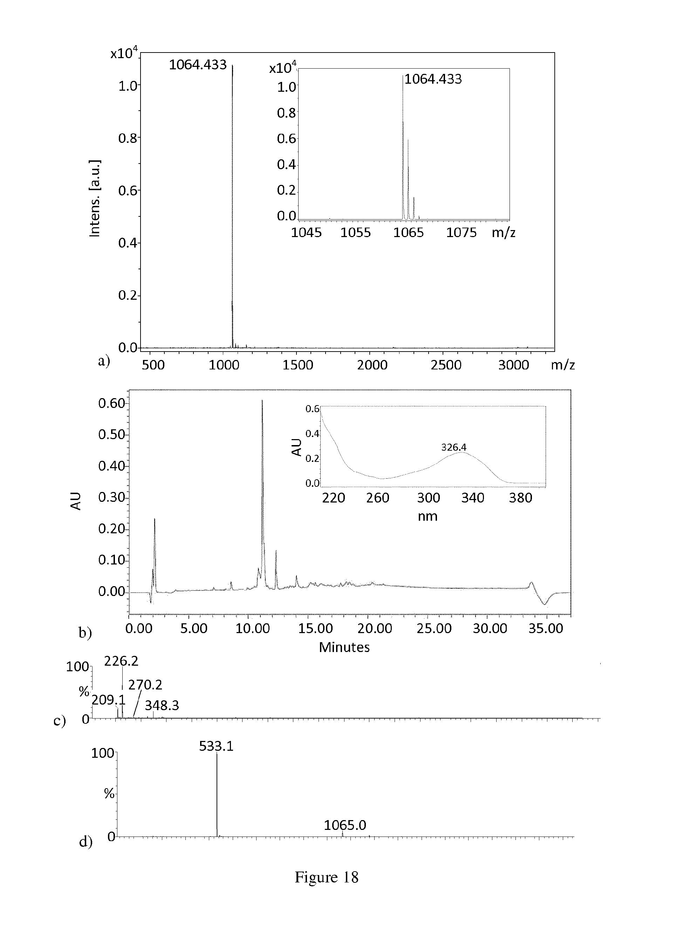

Figure 18: Characterization of compounds **9a** and **21c.** a) MALDI-TOF spectrum of **21c -** MALDI-TOF matrix: CHCA - Calculated for C₄₈H₆₉N₁₅O₁₃ [M+H]⁺ 1064.5, found 1064.4. b) RP-HPLC Analysis, **21c** RT=11.2 min. and **9a** RT= 12.3 min. Buffer A H₂O-0.05% TFA / Buffer B 0.05% TFA CH₃CN/water (4/1) column C18 Nucleosil 100 Å 5 µm (2.1x250 mm) with a linear gradient 0-100% B in 30 minutes (1.0 mL/min, detection from 210 to 400 nm). c) ESI spectrum of **9a** calculated for C₁₆H₂₇N₃O₄ [M+Na]⁺ 348.2, found 348.3 ; calculated for [M+H-Boc]⁺ 226.1, found 226.2. d) ESI spectrum of **21c** calculated for C₄₈H₆₉N₁₅O₁₃ [M+H]⁺ 1064.5, found 1065.0 ; calculated for [M+2H]⁺/2 532.7, found 533.1.

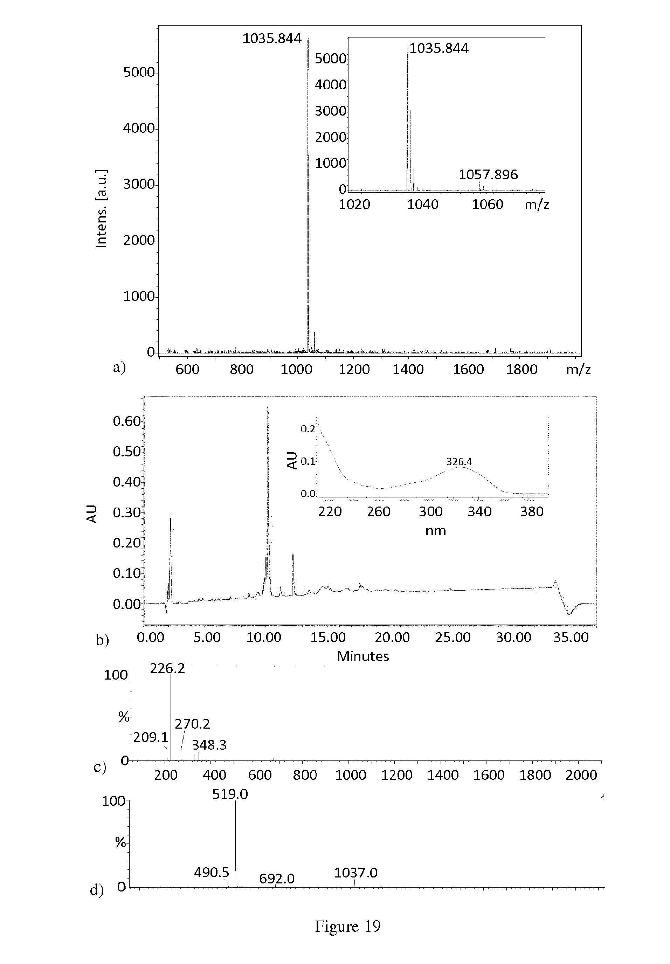

Figure 19: Characterization of compounds **9b** and **21a**. a) MALDI-TOF spectrum of **21a** - MALDI-TOF matrix: CHCA - Calculated for C₄₆H₆₅N₁₅O₁₃ [M+H]⁺ 1036.5, found 1035.8. b) RP-HPLC Analysis, **21a** RT=10.1 min. and **9b** RT= 12.1 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm). c) ESI spectrum of **9b** calculated for C₁₆H₂₇N₃O₄ [M+Na]⁺ 348.2, found 348.3 ; calculated for [M+H-Boc]⁺ 226.1, found 226.2. d) ESI spectrum of **21a** calculated for C₄₆H₆₅N₁₅O₁₃ [M+H]⁺ 1036.5, found 1037.0 ; calculated for [M+2H]⁺/2 518.7, found 519.0.

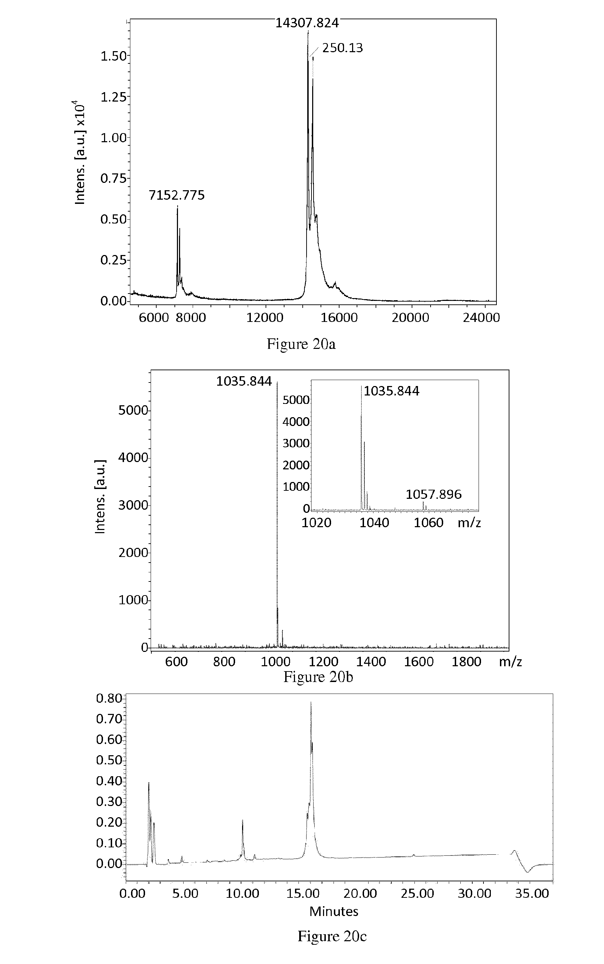

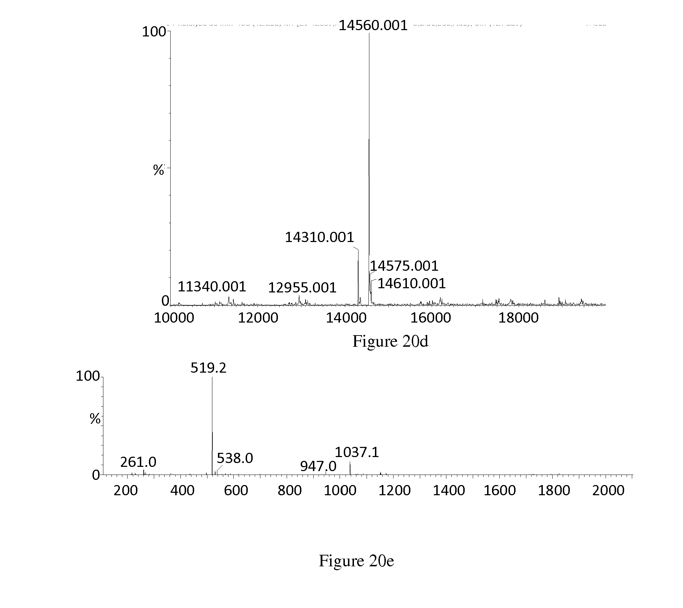

Figure 20: Characterization of compounds **10** and **21a**. a) MALDI-TOF spectrum of **10 -** MALDI-TOF matrix: DHB. b) MALDI-TOF spectrum of **21a** - MALDI-TOF matrix: CHCA - Calculated for C₄₆H₆₅N₁₅O₁₃ [M+H]⁺ 1036.5, found 1035.8. c) RP-HPLC Analysis, **21a** RT=10.3 min. and 10 RT= 16.0 min. Buffer A H₂O-0.05% TFA / Buffer B 0.05% TFA CH₃CN/water (4/1) column C18 Nucleosil 100 Å 5 µm (2.1x250 mm) with a linear gradient 0-100% B in 30 minutes (1.0 mL/min, detection from 210 to 400 nm). d) ESI spectrum of 10. e) ESI spectrum of **21a** calculated for C₄₆H₆₅N₁₅O₁₃ [M+H]⁺ 1036.5, found 1037.1 ; calculated for [M+2H]⁺/2 518.7, found 519.2.

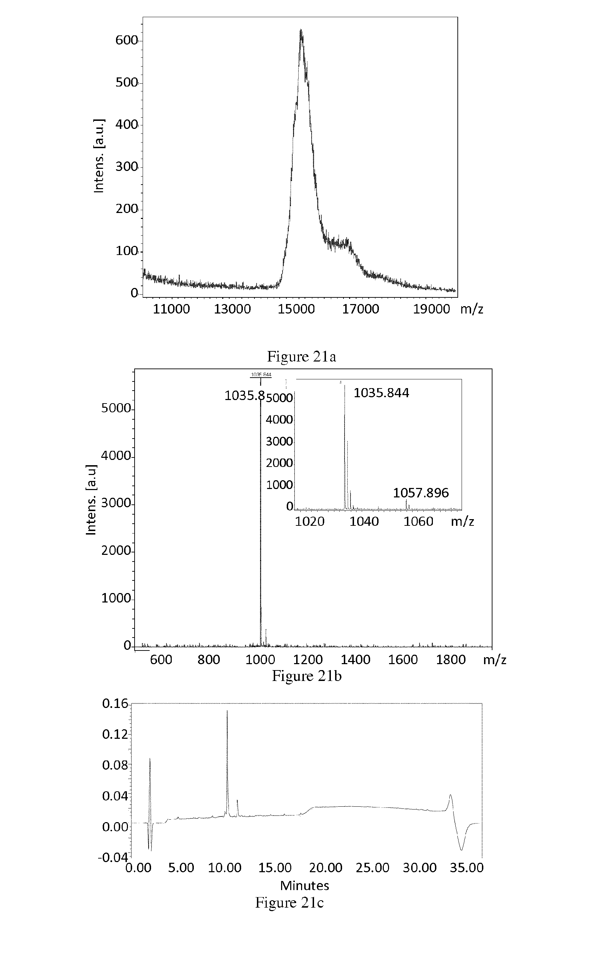

Figure 21: Characterization of compounds **22** and **21a.** a) MALDI-TOF spectrum of **10 -** MALDI-TOF matrix: DHB. b) MALDI-TOF spectrum of **21a -** MALDI-TOF matrix: CHCA - Calculated for C₄₆H₆₅N₁₅O₁₃ [M+H]⁺ 1036.5, found 1035.8. c) RP-HPLC Analysis, **21a** RT=10.1 min. and **22** RT= 19.5 min. Buffer A H₂O-0.05% TFA / Buffer B 0.05% TFA CH₃CN/water (4/1) column C3 Zorbax 300 SB, 3.5 µm (4.6×150 mm), 50°C, with a linear gradient 0-100%B in 30 minutes (1.0 mL/min, detection from 210 to 400 nm). d) ESI spectrum of **21a** calculated for C₄₆H₆₅N₁₅O₁₃ [M+H]⁺ 1036.5, found 1037.1 ; calculated for [M+2H]⁺/2 518.7, found 519.2.
Figure 22: Two previously described strategies for the labeling of binding molecules.
Figure 23: Tag release kinetics for compounds **5, 16a, 16b, 16c** and **16d.**

### Summary of the invention

The first object of the present invention is a label compound (a label) of the following formula: 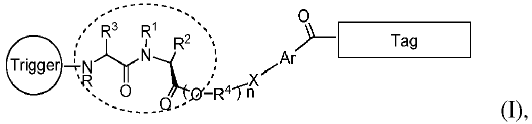 wherein:
- the tag and trigger moieties are as defined below,
- n is 0 or 1,
- Ar is an aromatic group,
- R is selected in the group consisting of a hydrogen atom, a saturated or unsaturated, linear or branched, alkyl group, optionally interrupted by at least one heteroatom selected in the group consisting of oxygen O, nitrogen N, and sulfur S atoms, and optionally substituted with at least one group selected in the group consisting of a hydroxy group, an alkoxy group, an amino NH₂ group, a nitro NO₂ group, a carboxylic acid COOH group,
- R¹ is a hydrogen atom and R² is selected in the group consisting of a hydrogen atom and a C1-C4 alkyl group, optionally substituted with a phenyl group; alternatively, R¹ and R² may form together and with the nitrogen and carbon atoms to which they are respectively linked a five membered ring; in particular, -R¹-R²- can be a -(CH₂)₃- group.
- R³ is a chemical group able to link, preferably to covalently link, a binding molecule. Preferably, R³ is of the formula -(CH₂)ₘ-CH₂-FG, wherein m is 0, 1, 2 or 3 and FG is a functional group that is able to react with a chemical group of a binding molecule.
- R⁴ is a C1-C4 alkyl group,
- X is selected in the group consisting of oxygen O, NH and sulfur S.

Preferably, FG is chosen in the group consisting of an amino group (NH₂), a carboxyl group (COOH), an ester, an acyl halide group, such as an acyl chloride group (COCl), an anhydride group, and a derivative thereof, such as an activated or protected derivative thereof.
In a first embodiment, FG is an amino group and can link to a binding molecule through a urea linker, for instance by transformation into a N-succinimidyl carbamate (NSC) and subsequent reaction with an amino group of the binding molecule.
In a second embodiment, FG is an activated ester and can link to a binding molecule through an amide linker, for instance by reaction with an amino group of the molecule.
In a third embodiment, FG is a carboxyl group (COOH), an ester, an acyl halide group such as an acyl chloride group (COCl), or an anhydride group, and can link to a binding molecule through an alkyldiamide linker, for instance by transformation into a N-succinimidyl carboxylic ester and subsequent reaction with an amino group of the molecule. An alkyldiamide linker is a linker of the formula 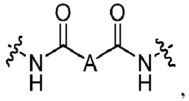 wherein A is an alkyl group and the bonds that are interrupted by a zigzag are the bonds linking the linker to the label and to the binding molecule.

An activated derivative of a chemical functional group is a derivative of said functional group that is a good leaving group. For instance, activated esters may be formed by reaction of a carboxylic acid with hydroxysuccinimide or hydroxybenzotriazole.
A protected derivative of a chemical functional group is a derivative of said functional group comprising a protecting group.
Among protecting groups of an amino functional group may be cited carbamates such as 2-trimethylsilylethyl carbamate (Teoc), 1-(1-adamantyl)-1-methylethyl carbamate (Adpoc), 1-methyl-1-(4-biphenyl)ethyl carbamate (Bpoc), *tert*-butyloxycarbonyl (Boc), 1-(3,5-di-t-butylphenyl)-1-methylethyl carbamate (t-Bumeoc), 1-adamantyl carbamate (Adoc), p-methoxybenzylcarbamate (MeOZ), 9-anthrylmethylcarbamate, diphenylmethylcarbamate, 9-fluorenylmethylcarbamate (Fmoc), 9-(2-Sulfo) fluoroenylmethylcarbamate, 9-(2,7-dibromo)fluorenylmethylcarbamate, 2,7-di-t-butyl-[9-(10,10-dioxo-10,10,10,10-tetrahydrothioxanthyl)]methylcarbamate (DBD-Tmoc), 2-(*N,N-*dicyclohexylcarboxamido)ethylcarbamate, 2-phosphonioethylcarbamate (Peoc), 2-phenylethylcarbamate, benzylcarbamate (Cbz), allylcarbamate (Alloc), 1-isopropylallylcarbamate (Ipaoc), 4-nitrocinnamylcarbamate (Noc), 8-quinolylcarbamate, acetyl (Ac) group, benzoyl (Bz) group, benzyl (Bn) group, *p*-methoxybenzyl (PMB) group, 3,4-dimethoxybenzyl (DMPM) group, *p*-methoxyphenyl (PMP) group, *N*-Phtalimide group, and tosyl (Ts) group.

Among protecting groups of a carboxyl functional group may be cited methyl esters, benzyl esters, *tert*-butyl esters, silyl esters, orthoesters and oxazolines.

One of ordinary skill in the art is able to select the appropriate activated and/ or protected derivatives of the functional group, depending on the structure of the label and the binding molecule.

The atoms inside the dotted-line ovoid in structure (I) form the dipeptide moiety.

"Detection" of a molecule of interest (or target molecule) refers in the present invention to detection of its presence, and/or quantification of the (absolute or relative) amount of molecule of interest in the sample. Preferably, "detection" of the molecule includes quantification of the amount of molecule in the sample.
A "stimulus" refers in the present invention to any event suitable for activating the trigger, such as a photochemical stress, a chemical reaction, and/or a biochemical process. For instance, the stimulus may be an irradiation or the digestion by an enzyme.
An "alkyl" group refers in the present invention to a saturated or unsaturated, preferably saturated, linear or branched, hydrocarbon group comprising from 1 to 8 carbon atoms (C1-C8 alkyl). Preferably, the alkyl groups comprises from 1 to 4 carbon atoms (C1-C4 alkyl). A C1-C4 alkyl group can in particular be a methyl, an ethyl, a propyl, an isopropyl, a butyl, an isobutyl, a *sec*-butyl or a *tert*-butyl group.
In a specific embodiment, n is 1 and R⁴ is selected among ethyl, propyl and butyl groups. According to the present invention, an alkyl group is preferably unsubstituted. In an embodiment, an alkyl group may be substituted by a phenyl group. In particular, the alkyl group may be a benzyl group (Bn).
An "alkoxy" group refers to an alkyl group as defined above linked to the rest of the molecule through an oxygen O atom.
An "aromatic" group refers to a hydrocarbon group comprising a hydrocarbon aromatic ring, optionally interrupted by at least one heteroatom such as an oxygen O, nitrogen N or sulfur S atom, preferably one O atom. The aromatic ring may be monocyclic or polycyclic, for instance it can be a phenyl, a naphthalenyl, an anthracenyl or a benzopyranyl group. Ar may be for instance formed from a phenolic derivative, for instance 4-hydroxybenzoic acid or 4-hydroxycinnamic acid, or a 7-hydroxycoumarine derivative, for instance 7-hydroxycoumarine-3-carboxylic acid, 7-hydroxy-4-methyl-3-coumarinyl acetic acid or 7-hydroxycoumarinyl-4-acetic acid. Thus, the X-Ar-C(=O) moiety may be selected among

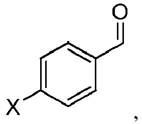

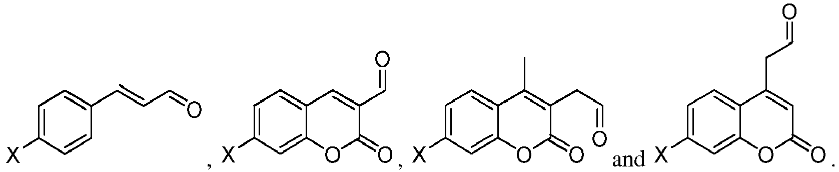

The label of the present invention is appropriate to link, in particular to covalently link, a binding molecule via the R³ moiety, to form a labeled molecule (or conjugate) as described below. In an embodiment, the binding molecule simultaneously links to several, for instance 2, 3, or more, labels.

The binding molecule is preferably linked to the label through an amino moiety, in particular an amino side-chain of a lysine, of the molecule. Alternatively, the molecule may be linked to the conjugate through a non-amino moiety, such as a hydroxyl OH or a thiol SH moiety, of the binding molecule.

The lateral position of the trigger moiety affords the possibility to easily modify its chemical structure, preferably without affecting the rest of the label structure, in particular the tag moiety, and thus the possibility to easily change the stimulus involved to release the tag moiety.

According to the invention, the activation of the trigger of the label and the cleavage between the trigger and the NR group through which the trigger moiety is linked to the rest of the label preferably induce an intramolecular cyclization of the dipeptide moiety into a diketopiperazine, ending with release of the Tag-C(=O)-Ar-X-(R⁴-O)ₙ-H compound.

Another object of the invention is a process for the preparation of a label according to the invention.

Other objects of the invention are a molecule labeled with a label according to the invention (a conjugate), and a method for preparing said conjugate.

Another object of the invention is a method for detecting a target molecule of interest in a sample, using a conjugate according to the invention.

Another object of the invention is a method of determining a risk of, or of detecting the predisposition to, or the presence of, a specific pathology in a subject, comprising detecting a target molecule specific of said pathology with at least a conjugate according to the invention.

Another object of the invention is a method for determining a target molecule map, comprising the use of at least one label or conjugate according to the invention.

Another object of the invention is a method for detecting at least one target molecule in a sample in an immuno-sensing assay, comprising the use of at least one label or conjugate according to the invention.

Another object of the invention is a composition of matter comprising a solid substrate, a compound bound to the solid substrate, wherein the compound is able to link at least one target molecule, the target molecule linked to the compound, and a conjugate of the invention which binds to the target molecule concurrently to the compound, wherein the conjugate is bound to the target molecule.

Another object of the invention is a kit for detecting at least one target molecule in a sample.

### Description of the invention

### Description of the constituents of the label

The trigger moiety is any chemical group that is cleaved when subjected to a stimulus. The stimulus may be for instance a photochemical stress, a chemical reaction (such as acid, basic or metallic) or a biochemical process. In particular, the trigger can be a photocleavable moiety, for which an appropriate stimulus is photoirradiation. Among the photocleavable moieties to be used in the present invention may be cited the 3'-nitrophenylpropyloxycarbonyl NPPOC 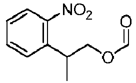 and 2-nitrobenzyl 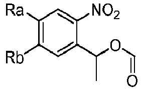 groups. Said groups may be used as protecting groups of an amine, via a bonding between the carbon atom of the C=O of the protecting group and the amine group. Said protecting groups are preferably introduced at the N-terminus of the dipeptide. Ra is selected in the group consisting of a hydrogen atom and an alkoxy group, such as a methoxy group. Rb is selected in the group consisting of a halogen atom, such as a chlorine atom, a hydroxyl group, an alkoxy group, such as a methoxy group, an amino NH₂ group and a piperazine 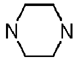 group. The NPPOC trigger can be photocleaved at a wavelength of about 365 nm. The 2-nitrobenzyl trigger can be photocleaved at a wavelength comprised between about 270 and about 370 nm, depending on the Ra and Rb substituents.
Activation of the trigger moiety with the stimulus produces an intermediate, primary (when R is H) or secondary, amine. Said amine is preferably nucleophilic. Said amine will launch an intramolecular cyclization leading to a diketopiperazine group and release of the Tag-C(=O)-Ar-X-(R⁴-O)ₙ-H compound.
In the present invention, the term "about" refers to a range ± 10% of the recited value.

The tag is any group of atoms susceptible, after release from the conjugate, optionally under the form of a compound comprising said tag, to be identified and/or detected by a technique of analysis. The compound released after activation of the trigger and the intramolecular cyclization of the diketopiperazine is preferably Tag-C(=O)-Ar-X-(R⁴-O)ₙ-H.
For instance, the tag may be a peptide, a molecule marked with a fluorescent and/or phosphorescent agent, a lipid, a protein, a drug, a nucleic acid such as DNA or RNA, a metallic compound or a ligand.
Detection of the tag or of the compound comprising said tag may be effected by any technique, for instance with a scanner, a Raman spectrometer, a mass spectrometer, a confocal spectrometer, an ultraviolet (UV) or infrared (IR) spectrometer, an NMR (Nuclear Magnetic Resonance) spectrometer, a liquid or gaseous chromatograph, or by capillary electrophoresis. Preferably, the tag is a tag of known mass. The tag, or the compound comprising said tag released from the conjugate, of known mass, can thus be detected by mass spectrometry, in particular by MALDI (Matrix-Assisted Laser Desorption/Ionisation) or ESI (Electrospray Ionization) mass spectrometry, or by liquid chromatography.
In a particular embodiment, the tag is a peptide of known mass, in particular a peptide of the sequence SEQ ID No: 1 Gly-Arg-Aa-Phe-Arg-Gly-Aa-Gly. Gly stands for glycine (G), Arg stands for arginine (R) and Phe stands for phenylalanine (F). The C-terminal group of said peptide moiety is preferably a primary amide. Amino acids Aa completing the backbone may be, independently of each other, selected in the group consisting of alanine (Ala, A), serine (Ser, S), valine (Val, V), asparagine (Asn, N), glutamine (Gln, Q), leucine (Leu, L) and proline (Pro, P). The 3 glycines in this sequence may be independently replaced by deuterium labeled glycines, for instance 2,2-d₂ glycine, allowing isotopic titration during the analysis. The mass of the Tag-C(=O)-Ar-X-(R⁴-O)ₙ-H compound of this specific embodiment is known and is comprised between 800 and 3000 Da, preferably between 800 and 1500 Da.

The dipeptide consists of two amino acids, the first and the second amino acids. The first amino acid can be a natural or non natural amino acid, or a derivative thereof. The first amino acid preferably displays an adjustable side-chain (R³) which allows a bond, preferably a specific bond, with the biomolecule. The first amino acid is preferably a natural amino acid comprising a side chain with an amino NH₂ group, such as lysine (m=3). Tuning of an acid function issued from a side-chain of aspartic acid (Asp) or glutamic acid (Glu) could also permit the biomolecule derivatization. Non natural amino acids such as 2,3-diaminopropionic acid (m=0) or 2,3-diaminobutyric acid (m=1) may also be considered. The second amino acid is selected among natural hydrophobic aliphatic amino acids, such as glycine (Gly, R¹=R²=H), alanine (Ala, R¹=H, R²=Me), valine (Val, R¹=H, R²=*i*-Pr), leucine (Leu, R¹=H, R²=*i*-Bu), isoleucine (Ile, R¹=H, R²=*s*-Bu) or proline (Pro, R¹=R²= -(CH₂)₃-) and aromatic amino acids, such as phenylalanine (Phe, R¹=H, R²=Bn).

Upon cleavage of the trigger moiety, the free primary or secondary NHR terminus of the dipeptide reacts with the C(=O)X function, such as ester function when X is an oxygen atom, of the dipeptide to trigger an intramolecular cyclization, the formation of a diketopiperazine, and release of the Tag-C(=O)-Ar-X-(R⁴-O)ₙ-H compound. The scheme below presents the cyclization for the specific case n=0 and R=H.

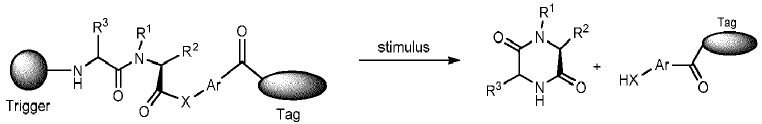

The link between the dipeptide and the tag-C(=O) moiety can be an arylester (n=0) when X is an oxygen atom, an arylamide (n=0) when X is a nitrogen atom or a thioester (n=0) when X is a sulfur atom. Ar may be for instance formed from a phenolic derivative, for instance 4-hydroxybenzoic acid or 4-hydroxycinnamic acid, or a 7-hydroxycoumarine derivative, for instance 7-hydroxycoumarine-3-carboxylic acid, 7-hydroxy-4-methyl-3-coumarinyl acetic acid or 7-hydroxycoumarinyl-4-acetic acid. Hydroxycoumarinyl moieties are especially useful in the present invention since they present a strong nucleofugal character and increase the rate of detection of peptides in MALDI and/or ESI mass spectrometry.

The binding molecule to which the label can link is a molecule able to link R³, preferably the FG group comprised in R³, as defined above. The binding molecule preferably comprises at least one amino (NH₂) group. The binding molecule can be an aptamer, including oligonucleic acid or peptide molecules that bind to a specific target molecule.
The binding molecule can thus be selected for instance in the group consisting of antibodies, such as monoclonal antibodies, native polyclonal antibodies and recombinant polyclonal antibodies, antigens, such as native antigens and recombinant antigens, haptenes, proteins, in particular ligands and receptors, glycoproteins, membrane proteins, peptides, saccharides, oligosaccharides, polysaccharides, peptidoglycans, lipids, nucleic acids such as DNA or RNA, oligonucleotides, aptamers, cells, such as cells comprised in a monolayer, and organic compounds such as drugs or hormones. One of ordinary skill in the art may easily adapt the binding molecule to the target molecule to detect.
Preferably, the binding molecule is an antibody or an antigen, in particular an antibody.

In the present invention, the term "antibody" shall include, without limitation, (a) an immunoglobulin molecule comprising two heavy chains and two light chains and which recognizes an antigen; (b) a polyclonal or monoclonal immunoglobulin molecule; and (c) a monovalent or divalent fragment thereof. Immunoglobulin molecules may derive from any of the commonly known classes, including but not limited to IgAl secretory IgA, IgG, IgE and IgM. IgG subclasses are well known to those in the art and include, but are not limited to, human IgGl, IgG2, IgG3 and IgG4. Antibodies can be both naturally occurring and non-naturally occurring. Furthermore, antibodies include chimeric antibodies, wholly synthetic antibodies, single chain antibodies, diabodies, single-chain antibodies, and fragments thereof. Antibodies may be human or nonhuman, humanized or not. Antibody fragments include, without limitation, Fab fragments, Fv fragments and other antigen-binding fragments.

### Preparation of a label according to the invention

The label according to the invention may be prepared by different processes depending on n=0 or n=1.

When n=0, the label compound can be prepared according to the following steps:
- step 1: preparation of a dipeptide (as depicted by the atoms inside the dotted-line ovoid in formula (I)), wherein the terminal functional group of the side-chain R³ may be protected, for instance with a *tert*-butyloxycarbonyl (Boc) protecting group when said functional group is an amine moiety,
- step 2: coupling of the dipeptide with the trigger as defined above,
- step 3: coupling of the compound obtained in step 2 with Tag-C(=O)-Ar-X-H,
- step 4: optionally deprotecting the functional group of the side-chain of the dipeptide, and
- step 5: optionally chemically activating or modifying the functional group of the dipeptide side-chain.

### Step 1:

Step 1 is preferably performed by peptidic coupling of the two amino acids according to methods well known in the art. More specifically, the dipeptide can be produced using a peptide synthesizer with standard solid-phase synthesis approaches (Fmoc/t-butyl or Boc/benzyl chemistry, as described in Merrifield R.B. Science 1986 232, 341-347). Preferably, the coupling is performed using Diisopropylcarbodiimide DIC (in particular 1 equivalent) as coupling agent and N-hydroxysuccinimide NHS (in particular 1 equivalent) as activator with N,N-diisopropylethylamine DIEA (in particular 2 equivalents) as base. The coupling may be performed with amino acids at least partially protected, for instance the terminal NH₂ moiety may be protected with a Fmoc (Fluorenylmethyloxycarbonyl) group. In such a case, step 1 may comprise a deprotection of the Fmoc group with piperidine.

### Step 2:

Step 2 consists in reacting the *N*-terminal amine group of the dipeptide obtained in step 1 with a reagent comprising the trigger moiety, to create a bond between the dipeptide and the trigger moiety. The reaction may be performed by any method known in the art to create said bond. Preferably, the reaction is performed by first contacting a trigger acyl halide with *N-*hydroxysuccinimide NHS at 0°C in a dichloromethane-pyridine mixture, for instance for 1h, to form the activated reagent comprising the trigger moiety. The obtained reagent is then preferably added to a solution of the dipeptide obtained in step 1 in an organic solvent, such as dichloromethane DCM.

### Step 3:

Coupling between the trigger-dipeptide obtained in step 2 and a Tag-C(=O)-Ar-X-H compound may be performed by any method known in the art. Step 3 is preferably performed with N,N'-Diisopropylcarbodiimide DIC (in particular 1.5 equivalent) and N-hydroxybenzotriazole HOBt (in particular 1.5 equivalent) as activator using DIEA (in particular 5 equivalents) as base in an organic solvent, such as dimethylformamide DMF.
The reagent Tag-C(=O)-Ar-X-H may be prepared by any method known in the art. It is preferably prepared according to the following process: the tag is prepared by solid phase synthesis, for instance using Fmoc/*tert*-butyl strategy on a Novasyn TGR resin with an INTAVIS synthesizer. Peptide elongations are carried out using for instance 5 equivalents of amino acids, 4.5 equivalents of coupling agent *O*-Benzotriazole-*N,N,N',N'*-tetramethyl-uronium-hexafluoro-phosphate HBTU and 10 equivalents of DIEA as base.

The *N*-terminal position of the peptide (tag), optionally linked to the solid phase resin, reacts with the HOOC-Ar-X-H compound, such as 7-hydroxycoumarin-4-acetic acid. The reaction may be performed in presence of 5 equivalents of HOOC-Ar-X-H, 5 equivalents of HOBt and 5 equivalents of DIC as coupling agent. If necessary, final deprotection and/or resin cleavage are carried out, for instance with trifluoroacetic acid TFA.

### Step 4:

Step 4 is the optional deprotection of the functional group (FG) of the side chain of the dipeptide. The conditions of step 4 are adapted by anyone of ordinary skill in the art depending on the protecting group and the structure of the compound. For instance, if the protected functional group is an amine group protected with a *tert*-butyloxycarbonyl Boc protecting group, step 4 may be carried out in a dichloromethane DCM trifluoroacetic acid TFA (1:1) mixture, at 0°C, for 2 hours.

### Step 5:

Step 5 consists in chemically modifying or activating the functional group (FG) of the side chain of the dipeptide in order to allow or favor further reaction with the binding molecule. When the functional group (FG) is a primary or secondary amine, the latter can be for instance transformed into a N-hydroxysuccinimidyl carbonyl, in order to form with the lysine amino acid of binding molecules an urea moiety. Step 5 can be performed by implementing classical chemical reactions that are well known by one of ordinary skill in the art.

When n=1, label compounds can be prepared according to the following steps:
- step 1: preparation of a HOOC-Ar-X-R⁴-OH compound, wherein Ar, X and R⁴ are as defined above,
- step 2: coupling of an amino acid with the trigger as defined above, wherein the terminal functional group of the side-chain R³ may be protected, for instance with a *tert*-butyloxycarbonyl (Boc) protecting group when said functional group is an amine moiety,
- step 3: preparation of amine label compound Tag-C(=O)-Ar-X-R⁴-O-dipeptide-trigger.
- step 4: optionally chemically activating or modifying the functional group of the dipeptide side-chain.

In embodiments of the invention, step 3 may be replaced with step 3', wherein step 3' comprises:
- step 3'a: preparation of Tag-C(=O)-Ar-X-R⁴-O-Pro, wherein R⁴ is preferably an ethyl, a propyl or a butyl group, and Pro is a proline,
- step 3'b: coupling of the molecule obtained in step 2 with Tag-C(=O)-Ar-X-R⁴-O-Pro obtained in step 3' a, and
- step 3' c: optionally deprotecting the functional group of the side-chain of the dipeptide.

### Step 1:

Step 1 consists in preparing modified aromatic groups with a spacer R⁴, wherein R⁴ is preferably an ethyl, propyl or butyl group. The preparation may be performed by any appropriate method known in the art. Preferably, the preparation may involve a step of alkylation of a hydroxyl group present on the starting aromatic compound, for instance alkylation of the hydroxyl group of 7-hydroxycoumarin-4-acetic acid.

### Step 2:

Step 2 consists in reacting the *N*-terminal amine group of an amino acid, preferably a protected amino acid, typically Nε-Boc-L-Lysine, with a reagent comprising the trigger moiety. The reaction may be performed by any method known in the art to create said bond. Preferably, the reaction is performed by first contacting a trigger acyl halide with *N*-hydroxysuccinimide NHS at 0°C in a dichloromethane-pyridine mixture, for instance for 1h, to form an activated reagent comprising the trigger moiety. The obtained reagent is then preferably added to a solution of the amino acid in an organic solvent, such as dichloromethane DCM.

### Step 3:

Step 3 is preferably performed as described below for step 3'a, except that Fmoc-proline is used instead of Boc-Proline. Fmoc basic deprotection of proline is preferably perfomed using piperidine (20%) in DMF on solid support. Then, coupling between the trigger-amino acid obtained in step 2 and free amine supported compound may be performed by any method known in the art, preferably with DIC (in particular 4.0 equivalent) and N-hydroxybenzotriazole HOBt (in particular 4.0 equivalent) as activator in an organic solvent, such as dimethylformamide DMF. Finally, deprotection and resin cleavage can be achieved, for instance with trifluoroacetic acid TFA, giving access to the Tag-C(=O)-Ar-X-R⁴-O-dipeptide-trigger compound.

### Step 4:

Step 4 consists in chemically modifying or activating the functional group (FG) of the side chain of the dipeptide in order to allow or favor further reaction with the binding molecule. When the functional group (FG) is a primary or secondary amine, the latter can be for instance transformed into a N-hydroxysuccinimidyl carbonyl, in order to form with the lysine amino acid of binding molecules an urea moiety. Step 4 can be performed by implementing classical chemical reactions that are well known by one of ordinary skill in the art.

### Step 3'a:

The tag may be prepared by any method known in the art. When the Tag is a peptide, it is preferably prepared by solid phase synthesis, for instance using Fmoc/*tert*-butyl strategy on a Novasyn TGR resin with an INTAVIS synthesizer or C.E.M. synthesizer. Peptide elongations are carried out using for instance 5 equivalents of amino acids, 4.5 equivalents of coupling agent *O*-Benzotriazole-*N,N,N',N'*-tetramethyl-uronium-hexafluoro-phosphate HBTU and 10 equivalents of DIEA as base.
Coupling of the tag with HOOC-Ar-X-R⁴-OH is then preferably performed by coupling said compound with an amino moiety of the tag, such as the *N*-terminal position of a peptidic tag. Said coupling can be performed for instance in presence of 5 equivalents of HOOC-Ar-X-R⁴-OH, 5 equivalents of HOBt and 5 equivalents of DIC as coupling agent. Coupling of Tag-CO-Ar-X-R⁴-OH with Proline can be performed for instance by acylation of the hydroxyl group with a derivate of Proline, such as Boc-Proline, Proline being of L or D configuration. Said coupling can be performed for instance using 8 equivalents of Boc-proline, 4 equivalents of coupling agent DIC and a catalytic amount of DMAP. If necessary, deprotection and resin cleavage can be carried out, for instance with trifluoroacetic acid TFA.

### Steps 3'b and 3'c:

Coupling between the trigger-amino acid obtained in step 2 and the Tag-C(=O)-Ar-X-R⁴-O-Pro obtained in step 3' a may be performed by any method known in the art.
Step 3'b is preferably performed with EDC (in particular 1.3 equivalent) and N-hydroxybenzotriazole HOBt (in particular 1.3 equivalent) as activator using DIEA (in particular 4 equivalents) as base in an organic solvent mixture, such as dimethylformamide-dichloromethane DMF-DCM.
If necessary, final deprotection (step 3'c) and/or resin cleavage are carried out, for instance with trifluoroacetic acid TFA.

### Conjugates

Another object of the invention is a molecule linked to a label according to the invention, also named a conjugate. Said conjugate is in particular of the formula (II): 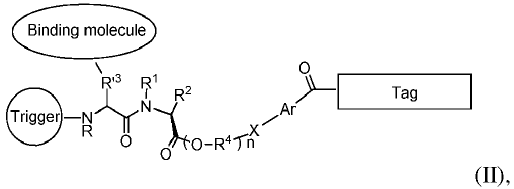 wherein
the tag, the trigger, the binding molecule, R, R¹, R², R⁴, X and n are as described above, and R'³ is a chemical group obtained from the reaction of the R³ group of the label and the binding molecule. In particular, R'³ is of the formula -(CH₂)ₘ-CH₂-FG', wherein FG' is a chemical group obtained from the reaction of the FG group of the label and the binding molecule.
R'³ preferably comprises, as link with the binding molecule, an urea, amide or alkyldiamide group. In particular, R'³ comprises an urea group. In particular, FG' is an urea, amide or alkyldiamide group, preferably an urea group.

Another object of the invention is a method for preparing a conjugate according to the invention.
The method for preparing a conjugate of the invention comprises contacting the binding molecule as defined above with at least one label of the invention.
In an embodiment, the method for preparing a conjugate of the invention comprises contacting at least one label of the invention with a sample comprising the binding molecule, in particular a biological sample comprising the binding molecule.

Contacting the label and the binding molecule is preferably performed at a temperature between about 0 and about 10°C, more preferably at a temperature between about 2 and about 5°C, in particular at a temperature about 4°C.
The method for preparing a conjugate preferably includes an additional step of purification of the conjugate. Said purification is preferably performed at a temperature between about 0 and about 10°C, more preferably at a temperature between about 2 and about 5°C, in particular at a temperature about 4°C.

### Applications

The conjugates of the invention are particularly useful for the detection of a target molecule, which binds to said conjugate, in a sample, preferably a biological sample.

The target molecule is a molecule of interest. It may display any chemical structure, provided that the conjugate and target molecule link, preferably specifically link, in the implemented experimental conditions. The target molecule can be chosen for instance in the group consisting of drugs, antibodies, such as monoclonal antibodies, native polyclonal antibodies and recombinant polyclonal antibodies, antigens, such as native antigens and recombinant antigens, haptenes, proteins, in particular ligands and receptors, glycoproteins, membrane proteins, peptides, saccharides, oligosaccharides, polysaccharides, peptidoglycans, lipids, nucleic acids such as DNA or RNA, oligonucleotides, aptamers, cells, such as cells comprised in a monolayer, and organic compounds such as drugs or hormones. Preferably, the target molecule is an antibody, a protein or an antigen, in particular a protein or an antibody.
The target molecule preferably links, or interacts with, in particular specifically links, or specifically interacts with, a conjugate of the invention. In particular, the target molecule links the binding molecule of the conjugate.

In an embodiment, the target molecule is an agent which presence in a biological sample is indicative of a risk of, a predisposition to, or the presence of, a pathology.

An object of the invention is a method for detecting at least one target molecule in a sample, preferably a biological sample, comprising the steps of:
(a) contacting the sample with at least one conjugate of the invention,
(b) removing any unbound conjugate from the sample,
(c) submitting the sample obtained after step (b) to the at least one appropriate stimulus to activate the conjugate(s), and
(d) detecting the release of the Tag-C(=O)-Ar-X-(R⁴-O)ₙ-H compounds by any appropriate analysis technique, preferably by MALDI mass spectrometry,
wherein the presence of the Tag-C(=O)-Ar-X-(R⁴-O)ₙ-H compounds indicates the presence of the corresponding target molecules in the sample.

The method for detecting at least one target molecule may include, before step (a), a step (a') of preparing the conjugate as described above.

In an embodiment, the method for detecting at least one target molecule comprises the use of at least one, preferably at least 2, more preferably from 5 to 25, conjugates of the invention, preferably comprising different Tag-C(=O)-Ar-X-(R⁴-O)ₙ moieties, to detect at least one, preferably at least 2, more preferably from 5 to 25, target molecules in the sample.
In an embodiment, the number of conjugates of the invention, preferably comprising different Tag-C(=O)-Ar-X-(R⁴-O)ₙ moieties, is equal to the number of target molecules to detect in the sample.

When several conjugates with different triggers are used, the activation of the different conjugates with the appropriate stimuli may be simultaneous and/or successive.
Step (c), or the activation of at least one conjugate, and step (d) may be performed simultaneously. For instance, if the analysis technique used to detect the release of the Tag-C(=O)-Ar-X-(R⁴-O)ₙ-H compounds is LASER MALDI, the LASER irradiation of the MALDI may be the appropriate stimulus to activate at least one conjugate.

Preferably, the presence of the Tag-C(=O)-Ar-X-(R⁴-O)ₙ-H compound in the sample may be detected by mass spectrometry, such as MALDI or ESI mass spectrometry, or by liquid chromatography. Matrix Assisted Laser Desorption/Ionization (MALDI) mass spectrometry has become a powerful tool in the field of biological researches and is used for the detection, identification and characterization of nucleic acids, peptides and proteins from complex mixtures.

In an embodiment, the trigger is a NPPOC group, and the irradiating wavelength is about 365 nm. In another embodiment, the trigger is a nitrobenzyl group, and the irradiating wavelength is between about 270 nm and 370 nm.

In a preferred embodiment, step (c) of the method for detecting at least one target molecule in a sample is performed at a pH comprised between 7 and 9, preferably around 8.

The method for detecting at least one target molecule in a biological sample and/or the conjugates of the invention may be used in medical diagnosis and/or clinical biology. In the context of the invention, a biological sample refers to a biological fluid, such as blood, plasma, serum, saliva, urine, etc., a biological tissue, or a part thereof, or cells. Advantageously, the method of detection of the invention may be applied directly on tissue sections. Among application fields may be cited genetic tests, biochemistry, immunochemistry, infectious immunology, microbiology and hematology. For instance, the invention may be used to study the distribution and/or the pharmacokinetics of a molecule of interest, such as a drug, in various biological samples.

Another object of the invention is a method of determining a risk of, or of detecting the predisposition to, or the presence of, a specific pathology in a subject, comprising detecting a target molecule specific of said pathology with at least a conjugate according to the invention. In particular, detection of said target molecule specific of a pathology may be performed with the method for detecting at least one target molecule in a biological sample described above. Examples of pathologies are cancers, autoimmune pathologies, congenital hypothyroidism, cardiological pathologies, phosphocalcic metabolism pathologies, bacteriology pathologies and neonatology pathologies. Among cancers may be cited in particular glioblastomas, (promyelocytary) leukemias, cancers of the prostate, the ovaries, the lungs, the breasts, the digestive tract, in particular of the liver, of the pancreas, of the head and of the neck, of the colon, of the bladder, non-Hodgkin lymphomas and melanomas.

The method for detecting a target molecule described above may be applied for the determination of at least one target molecule map (i.e. distribution) in a tissue section, and/or for the detection of said at least one target molecule in a sample in a multiplex immuno-sensing assay.

### Determination of a target molecule map

The conjugates of the invention may be used in a method for determining at least one target molecule map in a sample, preferably in a tissue section, for instance as described in WO 2007/000669 or in Lemaire et al. J. Prot. Res. 2007, 6, 2057.

Preferably, the method for determining a target molecule map comprises the use of at least one conjugate comprising a photocleavable trigger moiety, preferably all the conjugates involved in said method comprise a photocleavable trigger moiety.

According to the invention, a "tissue section" preferably has the following properties: it may be frozen or paraffin-embedded, its thickness is preferably in the order of a mammalian cell diameter, thus comprised between 5 and 20 µm (micrometers). In the case of a frozen section that was obtained from a frozen tissue using a cryostat, OCT (optimal cutting temperature polymer) is preferably used only to fix the tissue but the frozen tissue is not embedded in OCT, so that tissue sections were not brought into contact with OCT. The tissue section may then be transferred on a MALDI plate composed of any material suitable for further MALDI analysis, including metals, inorganic or organic materials, such as gold, steel, glass fiber, glass, nylon 6/6, silicon, plastic, polyethylene, polypropylene, polyamide, polyvinylidenedifluoride or a glass slice of any thickness coated with conductive metal keeping transparency properties such as nickel or ITO (indium tin oxide).

In an embodiment, the method for determining a target molecule map in a tissue section according to the invention comprises the steps of:
(a) hybridizing said tissue section with at least one conjugate according to the invention, suitable for binding the target molecule,
(b) spraying a matrix,
(c) scanning the tissue section surface with a MALDI mass spectrometer, wherein the MALDI laser is preferably used both to release the Tag-C(=O)-Ar-X-(R⁴-O)ₙ-H compound and to induce the sample ionization, and wherein the resulting data is saved; and
(d) analyzing the obtained data in the molecular mass window(s) of each distinct Tag-C(=O)-Ar-X-(R₄-O)ₙ-H compound to create as many maps of the tissue section as the number of distinct studied target molecules.

According to the invention, "hybridizing said tissue section with at least one conjugate" refers to a reaction in which the tissue section and the conjugate(s) are brought into contact in such conditions that the conjugate is able to bind specifically to its target molecule in the tissue section. Depending on the nature of the conjugate and the target molecule, well-known hybridization protocols are available to one skilled in the art.
By "matrix" is meant any material that, when mixed with the analyte, generates crystalline matrix-embedded analyte molecules that are successfully desorbed by laser irradiation and ionized from the solid phase crystals into the gaseous or vapour phase and accelerated as molecular ions. Commonly used MALDI-MS matrices are generally small, acidic chemicals absorbing at the laser wavelength, including nicotinic acid, cinnamic acid, 2,5-dihydroxybenzoic acid (2,5-DHB), α-cyano-4-hydroxycinnamic acid (CHCA), 3,5-dimethoxy-4-hydroxycinnamic acid (sinapinic acid or SA), 3-methoxy-4-hydroxycinnamic acid (ferulic acid), 3,4-dihydroxycinnamic acid (caffeic acid), 2-(4- hydroxyphenylazo)benzoic acid (HABA), 3-hydroxy picolinic acid (HPA), 2,4,6- trihydroxy acetophenone (THAP) and 2-amino-4-methyl-5-nitropyridine. Protocols for the preparation of these matrices are well-known in the art, and most of these matrices are commercially available. Current commonly used matrices for peptide/protein analysis include α-cyano-4-hydroxycinnamic acid (CHCA), 2,5-dihydroxybenzoic acid (2,5-DHB) and sinapinic acid (SA). DNPH is 2,4-dinitrophenylhydrazine and is used for aldehydes and ketones detection.

### Multiplex immuno-sensing assay

The conjugates of the invention may be used in a method for detecting at least one target molecule in a sample in an immuno-sensing assay, for instance as described in WO 2007/062105.

An object of the invention is a method for detecting at least one target molecule in a sample in an immuno-sensing assay, comprising the use of at least one label or conjugate according to the invention.

The immuno-sensing assay may be indirect, direct (sandwich), or competition immuno-sensing assay.
For instance, the method for detecting at least one target molecule of the invention can be used for the dosing of the TSH (thyroid-stimulating hormone) and T4 (thyroxine T4) hormones, preferably via a sandwich direct immuno-sensing assay, preferably with monoclonal antibodies, for TSH, and a competition method for T4. Increased TSH and decreased T4 are actually clear signs of congenital hypothyroidism.

In a first embodiment, the method for detecting at least one target molecule in a sample in an immuno-sensing assay comprises the steps of:
(a) contacting the sample with a solid substrate having affixed thereto at least one compound able to link the at least one target molecule, wherein the contacting is performed under conditions which would permit the compound(s) to bind to the target molecule(s) if present in the sample,
(b) removing any unbound sample from the solid substrate,
(c) contacting the solid substrate obtained in step (a) with at least one conjugate of the invention, wherein the contacting is performed under conditions which would permit the conjugate(s) to bind to the target molecule(s) if present on the solid substrate,
(d) removing any unbound conjugate,
(e) submitting the sample obtained in step (c) to the appropriate stimulus (stimuli) to activate the conjugate(s), and
(f) detecting the release of the Tag-C(=O)-Ar-X-(R⁴-O)ₙ-H compound(s) by any appropriate analysis technique, preferably by MALDI mass spectrometry,
wherein the presence of the Tag-C(=O)-Ar-X-(R⁴-O)ₙ-H compound(s) indicates the presence of the corresponding target molecule(s) in the sample.

In another embodiment, for each target molecule, there is:
- at least one compound able to link the target molecule affixed to the solid substrate of step (a), and
- at least one conjugate which binds to the target molecule concurrently with the compound able to link the target molecule, and
the tag of the at least one conjugate has a different signal of detection, in particular a different mass, than that of the tag of the conjugates that bind any other target molecule.

In the present invention, having a different signal of detection preferably means that the detection signals are sufficiently different to be distinguishable when using the appropriate detection and/or analysis technique.

In a third embodiment, the method for detecting at least one target molecule in a sample in an immuno-sensing assay comprises the steps of:
(a) contacting the sample with a solid substrate which binds to the target molecule(s), wherein the contacting is performed under conditions which would permit the support to bind to the target molecule(s) if present in the sample,
(b) removing any unbound sample from the solid substrate,
(c) contacting the solid substrate obtained in step (a) with at least one conjugate of the invention that binds to the target molecule(s) concurrently with the support, wherein the contacting is performed under conditions which would permit the conjugate(s) to bind to the target molecule(s) if present on the solid substrate,
(d) removing any unbound conjugate,
(e) submitting the sample obtained in step (d) to the appropriate stimulus (stimuli) to activate the conjugate(s), and
(f) detecting the release of the Tag-C(=O)-Ar-X-(R⁴-O)ₙ-H compound(s) by any appropriate analysis technique, preferably by MALDI mass spectrometry,
wherein the presence of the Tag-C(=O)-Ar-X-(R⁴-O)ₙ-H compound(s) indicates the presence of the corresponding target molecule(s) in the sample.

In a fourth embodiment, the solid substrate of step (a) binds to several target molecules, and, for each target molecule, there is at least one conjugate which binds to the target molecule concurrently with the solid substrate, and the tag of the at least one conjugate has a different signal of detection, in particular a different mass, than that of the tag of the conjugates that bind any other target molecule.

An object of the invention is a composition of matter comprising:
(a) a solid substrate,
(b) a compound bound to the solid substrate, wherein the compound is able to link at least one target molecule,
(c) the target molecule linked to the compound, and
(d) a conjugate of the invention which binds to the target molecule concurrently to the compound, wherein the conjugate is bound to the target molecule.

In the present invention, the "solid substrate" shall mean any suitable medium present in the solid phase to which a compound able to link at least one target molecule may be affixed. In an embodiment, the solid substrate is glass, quartz, silicon, plastic, or gold. In another embodiment, the solid substrate comprises, preferably consists of, at least one insoluble polymer, such as agarose gel or polystyrene. The solid substrate can be for example in the form of a bead, a chip, or a well.
The "compound able to link at least one target molecule" is any compound able to specifically link, preferably to covalently link, said target molecule. Said compound is preferably an antigen or an antibody, in particular an antigen. The compound can be affixed to the solid substrate, for example, via a streptavidin-biotin link or via 1,3-dipolar cycloaddition.

An object of the invention is a kit for detecting a target molecule in a sample comprising:
(a) a solid substrate,
(b) a compound able to link a target molecule for affixing to the solid substrate,
(c) a conjugate of the invention that links the target molecule concurrently with the compound, and
(d) instructions for using the kit to detect the target molecule in the sample.

An object of the invention is a kit for detecting a target molecule in a sample comprising:
(a) a solid substrate affixed with a compound able to link the target molecule, and
(b) a conjugate of the invention, which conjugate binds the target molecule, and
(c) instructions for using the kit to detect the target molecule in the sample.

An object of the invention is a kit for detecting a target molecule in a sample comprising:
(a) a solid substrate which binds the target molecule,
(b) a conjugate of the invention, which conjugate binds the target molecule, and
(c) instructions for using the kit to detect the target molecule in the sample.

An object of the invention is a kit for detecting at least one target molecule in a sample comprising:
(a) a solid substrate,
(b) a plurality of compounds able to link target molecules for affixing to the solid substrate, wherein for each target molecule there is at least one compound able to link said target molecule,
(c) a plurality of conjugates of the invention, wherein for each target molecule there is at least one conjugate that links said target molecule concurrently with the compound, and wherein the tag of each conjugate has a different signal of detection, in particular a different mass, than that of the tag of the conjugates that bind any other target molecule, and
(d) instructions for using the kit to detect at least one target molecule in the sample.

An object of the invention is a kit for detecting at least one target molecule in a sample comprising:
(a) a solid substrate having affixed thereto a plurality of compounds able to link target molecules, wherein for each target molecule there is at least one compound able to link said target molecule,
(b) a plurality of conjugates of the invention, wherein for each target molecule there is at least one conjugate that links said target molecule concurrently with the compound, and wherein the tag of each conjugate has a different signal of detection, in particular a different mass, than that of the tag of the conjugates that bind any other target molecule, and
(c) instructions for using the kit to detect at least one target molecule in the sample.

A last object of the invention is a kit for detecting at least one target molecule in a sample comprising:
(a) a solid substrate which binds each of the target molecules,
(b) a plurality of conjugates of the invention, wherein for each target molecule there is at least one conjugate that links said target molecule and wherein the tag of each conjugate has a different signal of detection, in particular a different mass, than that of the tag of the conjugates that bind any other target molecule, and
(c) instructions for using the kit to detect at least one target molecule in the sample.

The examples below are provided only as illustrative, and not limitative, of the present invention.

### Examples

### ● For n=0

### Example 1: Preparation of a conjugate according to the invention

Solid phase synthesis has been achieved using Fmoc/*tert*-butyl strategy on a Novasyn TGR resin with an INTAVIS synthesizer. Peptide elongations have been carried out using 5 equivalents of amino acids, 4.5 equivalents of coupling agent *O*-Benzotriazole-*N,N,N',N'-*tetramethyl-uronium-hexafluoro-phosphate HBTU and 10 equivalents of DIEA as base. *N-*terminal position of the peptide resin reacts with 7-hydroxycoumarin-4-acetic acid (5 equivalents) with hydroxybenzotriazole (5 equivalents) and diisopropylcarbodiimide (5 equivalents) as coupling agent. Final deprotection and resin cleavage are carried out with trifluoroacetic acid TFA and tag peptides **1a-e** are obtained after purification with yields between 48 and 79 % (Step 1).
All compounds of the process have been purified by reverse phase liquid chromatography RP-HPLC on Nucleosil C18 column using a linear gradient water/acetonitrile containing 0.05% TFA. Compounds purity has been determined to be superior to 95% by RP-HPLC. All synthetic structures have been confirmed by MALDI-TOF mass spectrometry. NMR analysis of peptide **1a** has also been performed. PG= Protecting Group.

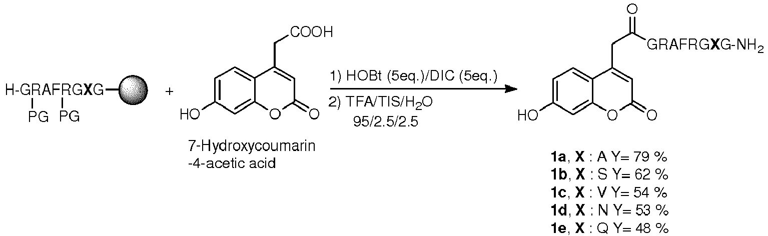

### Step 1:

*N*_{α}-Fmoc-*N*_{ε}-Boc-L-Lysine Lys is coupled to L-Proline Pro using DIC (1 equivalent) as coupling agent and NHS (1 equivalent) as activator with DIEA (2 equivalents) as base. Dipeptide 2 FmocLys(Boc)Pro is used without further purification for the next step. Fmoc deprotection with piperidine gives dipeptide Lys(Boc)Pro 3 with a 50 % yield after purification over the 2 steps.

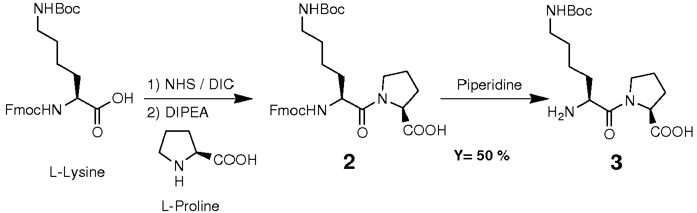

### Step 2:

The next step consists in protecting the *N*-terminal amine group of dipeptide Lys(Boc)Pro 3 with 2-(nitrophenyl)propyloxycarbonyl group. First 2-(nitrophenyl)propyloxycarbonyl chloride NPPOC reacts with NHS at 0°C in a dichloromethane-pyridine mixture for 1h00. The corresponding NPPOC-OSu is added dropwise to a dipeptide Lys(Boc)Pro **3** solution in dichloromethane DCM and stirred at room temperature for 18h00. After preparative RP-HPLC purification, dipeptide NPPOCLys(Boc)Pro **4** is isolated in 45 % yield.

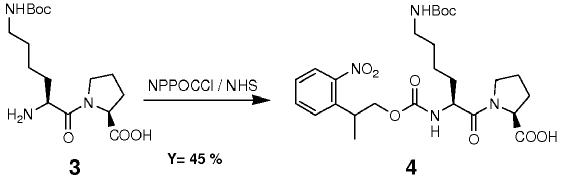

### Step 3:

Coupling between NPPOClys(Boc)Pro **4** and tag peptide **1a** is accomplished with DIC (1.5 equivalent) and HOBt (1.5 equivalent) as activator using DIEA (5 equivalents) as base in dimethylformamide DMF for 20 hours. Ester 5 is isolated after purification with a 46 % yield.

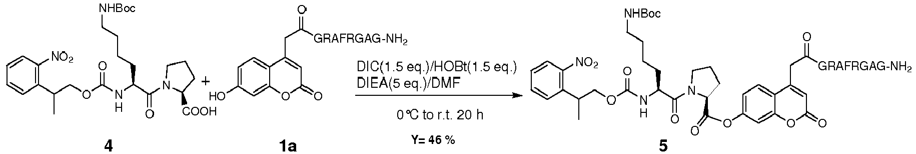

5 is characterized by MALDI-TOF and RP-HPLC, and results are presented on figure 1.

### Step 4:

Removal of *tert*-butyloxycarbonyl Boc protecting group of dipeptide ester 5 is carried out in a dichloromethane DCM trifluoroacetic acid TFA (1:1) mixture at 0°C for 2 hours. Purification leads to dipeptide amine ester **6** with a 49 % yield.
In order to prevent any photolytic side-reactions, all reactions with compounds bearing the protecting group NPPOC on N-terminal position have been carried out in a dark place.

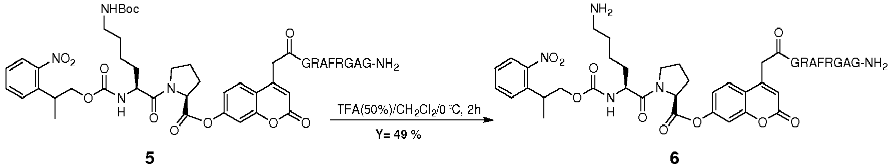

6 is characterized by MALDI-TOF and RP-HPLC, and results are presented on figure 2.

### Step 5:

Amine ester dipeptide 6 reacts with di(*N*-succinimidyl)carbonate DSC (10 equivalents) and a catalytic amount of triethylamine (0.5 equivalent) in DMF at room temperature for 30 minutes. *N*-hydroxysuccinimidyl carbonyl dipeptide 7 is isolated with a 98 % yield after purification.

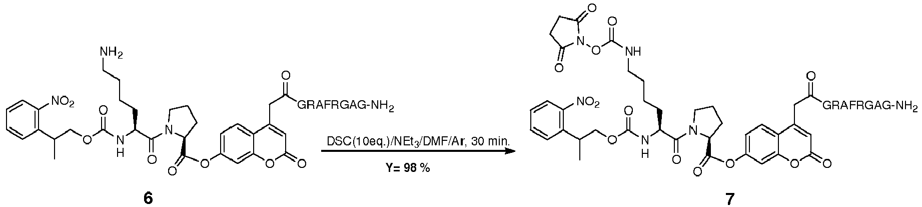

7 is characterized by MALDI-TOF and RP-HPLC, and results are presented on figure 3.

Analysis of compound 7 in MALDI-TOF mass spectrometry revealed isocyanate form was preponderant compared to the NSC form.

### Example 2: Biomolecule Labeling according to the invention

In order to validate the method proof of concept regarding the labeling with photocleavable tag peptide NSC **7,** lysozyme was chosen as binding molecule. Lysozyme is a globular protein composed of 129 amino acids having a molecular weight of 14.3 kDa and featuring 7 primary amine groups, and more accurately 6 lysines and a *N*-terminal amine group. Labeling of lysozyme with photocleavable tag peptide NSC **7** was investigated following the protocol described in Mhidia et al. Bioconjugate Chem. 2010, 21, 219-228. A 0.2 mM Lysozyme solution (1 equivalent) in PBS buffer (Phosphate Buffer Saline) at pH=7.4 was added to the photocleavable tag peptide NSC **7** (4 equivalents) at 4°C and in a dark place. RP-HPLC kinetic analysis showed no more conversion after 2 hours. Reaction medium analysis displayed that 80 % of lysozyme has been labeled by one or several photocleavable tag peptide **7.** Nevertheless chromatogram showed that, first not all NSC 7 compound has been consumed (peak at RT=18.8 min.) and, secondly a hydrolysis side reaction leading to amine ester dipeptide **6** (peak at RT=16.1 min.) occurred. MALDI-TOF mass spectrometry confirmed those observations. Indeed just a small quantity of lysozyme was detected [M+H]⁺ 14296 Da. The main product was lysozyme labeled with label **8** (p=1), characterized by a [M+H]⁺ mass of 15760 Da. Lysozyme has also been labeled with two labels **7** (p=2) defined by a [M+H]⁺ mass of 17213 Da and with three labels 7 (p=3) identified by a [M+H]⁺ mass of 18662 Da.

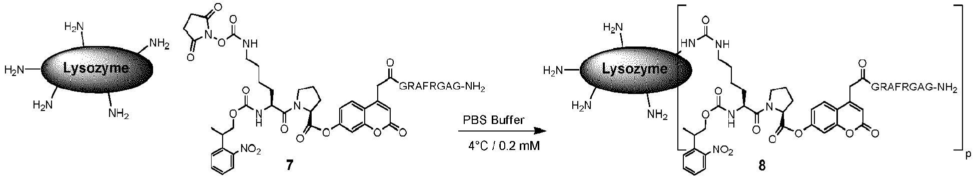

In order to remove any traces of hydrolyzed amine ester dipeptide **6** and unreacted photocleavable tag peptide NSC **7,** labeled lysozyme was purified by washing three times with phosphate buffer under centrifugation (RCF=3000) at 4°C on a Vivaspin cartridge (cut-off 10000) for 4h00.
**8** is characterized by MALDI-TOF and RP-HPLC, and results are presented on figure 4.

Same reaction has been achieved using only one equivalent of photocleavable tag peptide NSC **7,** the obtained product is **8a.** Kinetic rate determination revealed all compound **7** has been consumed after 30 minutes and 40 % of lysozyme has been labeled. MALDI-TOF mass spectrometry analysis showed that lysozyme has been partially labeled by one photocleavable tag peptide (p=1) characterized by a [M+H]⁺ mass of 15742 Da. **8a** is characterized by MALDI-TOF and RP-HPLC, and results are presented on figure 5.

### Example 3: Photocleavage of compound 5 and release of tag 1a

Photolabile NPPOC protecting group was cleaved by irradiating a 1 mM solution of tag peptide ester **5** in phosphate buffer at room temperature for 10 minutes with a 100 Watt UV lamp (λ=365 nm). RP-HPLC analysis proved the irradiation exposure leads to total disappearance of starting material NPPOC tag peptide ester 5 (RT=20.9 min., step 4) and emergence of two major products with retention times of RT=11.7 min. and RT=14.4 min. Mass spectrometry analysis by electrospray ionization ESI confirmed the product having RT=14.4 min. corresponded to diketopiperazine **9** with a [M+Na]⁺ mass of 348.3. ESI and MALDI-TOF analysis did also confirm that product characterized by RT=11.7 min. was tag peptide **1a**.

The following photocleavage step validated the invention proof of concept, i.e. the cleavage by UV irradiation of photolabile protecting group NPPOC of tag peptide **5** will generate the corresponding primary amine, which will induce instantly an intramolecular cyclization reaction leading to diketopiperazine **9** and above all release of tag peptide **1a**.

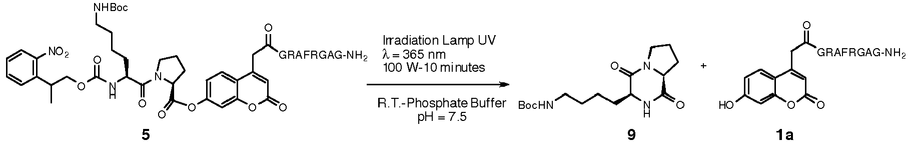

**1a** and **9** are characterized by RP-HPLC, ESI and MALDI-TOF (**1a**). Results are presented on figure 6.

### Example 4: Photocleavage of compound 8 and release of tag 1a

Irradiation at room temperature for 15 minutes with a 100 Watt UV lamp (λ=365 nm) at pH 7.5 of photocleavable tag peptide labeled lysozyme **8** leads to a change of chromatographic profile in RP-HPLC. Indeed a product with a retention time of RT=11.7 min. appeared. This product was confirmed to be the tag peptide **1a** after MALDI-TOF analysis. MALDI-TOF mass spectrometry analysis of the second product at RT=17.1 min. displayed a [M+H]⁺major mass of 14557.7 Da matching the diketopiperazine lysozyme 10. This result validates the proof of concept for the irradiation of photocleabable labeled lysozyme 8 leading to an intramolecular cyclization and the release of the tag peptide **1a**.

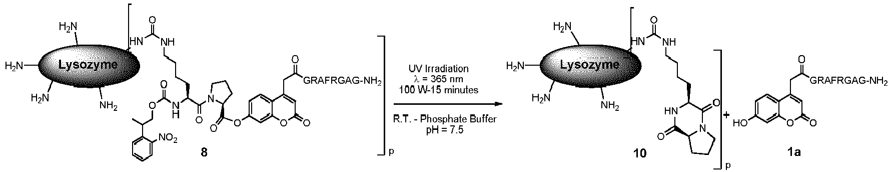

**1a** and 10 are characterized by RP-HPLC, and MALDI-TOF (**1a**). Results are presented on figure 7.

### ● For n=1

### Example 5: Preparation of a conjugate according to the invention

### Step 1:

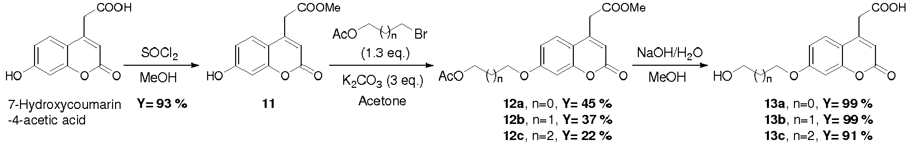

Hydroxy alkyl acids 13a-c were synthesized in 3 steps starting from 7-hydroxycoumarin-4-acetic acid. Starting material 7-hydroxycoumarin-4-acetic acid is first modified into methyl ester **11** by reaction with thionyl chloride in methanol with 93 % yield. Phenol moiety of methyl ester **11** is deprotonated by potassium carbonate (3 equivalents) in acetone and forms a phenol alkyl ether by reacting with bromo alkyl (1.3 equivalent) n=0, 1, 2. Corresponding phenol alkyl ethers **12a-c** were obtained after purification with yields between 22 and 45 %. Subsequent saponification of **12a-c** using sodium hydroxide in methanol gave hydroxy alkyl acids **13a-c** with yields comprised between 91 and 99 %.
**11:** C₁₂H₁₀O₅ [M+H]⁺ 235.1, found 235.1 ; RP-HPLC Analysis, RT=11.5 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm).
**12a:** C₁₆H₁₆O₇ [M+H]⁺ 321.1, found 321.2 ; RP-HPLC Analysis, RT=15.7 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm).
**12b:** C₁₇H₁₈O₇ [M+H]⁺ 335.1, found 335.3 ; RP-HPLC Analysis, RT=17.6 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm).
**12c:** C₁₈H₂₀O₇ [M+H]⁺ 349.1, found 349.2 ; RP-HPLC Analysis, RT=19.2 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm).
**13a:** MALDI-TOF: matrix DHB - Calculated for C₁₃H₁₂O₆ [M+H]⁺ 265.1, found 265.0; [M+Na]⁺ calculated for 287.0, found 287.0. RP-HPLC Analysis, RT=9.4 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm). ¹H NMR (300 MHz, DMF) δ 7.7 (d, *J* = 9.4 Hz, 1H), 7.1 - 6.9 (m, 2H), 6.4 (s, 1H), 4.2 (t, *J* = 4.5 Hz, 2H), 4.0 (s, 2H), 3.9 (t, *J* = 4.5 Hz, 2H), 3.6 (s, 2H). D) ¹³C NMR (75 MHz, DMF) δ 171.2, 136.1, 161.0, 156.1, 150.9, 127.3, 113.7, 113.4, 113.3, 102.0, 71.3, 60.6, 38.1.
13b: MALDI-TOF: matrix DHB - Calculated for C₁₄H₁₄O₆ [M+H]⁺ 279.1, found 279.1; [M+Na]⁺ calculated for 301.1, found 301.1. RP-HPLC Analysis, RT=10.8 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm). ¹H NMR (300 MHz, DMF) δ 7.7 (d, *J* = 9.4 Hz, 1H), 7.1 - 6.9 (m, 2H), 6.4 (s, 1H), 4.2 (t, *J* = 4.5 Hz, 2H), 4.0 (s, 2H), 3.9 (t, *J* = 4.5 Hz, 2H), 3.6 (s, 2H). D) ¹³C NMR (75 MHz, DMF) δ 171.2, 163.0, 161.0, 156.1, 150.9, 127.3, 113.7, 113.4, 113.3, 102.0, 71.3, 60.6, 38.1; 37.7.
13c: MALDI-TOF: matrix DHB - Calculated for C₁₅H₁₆O₆ [M+H]⁺ 293.1, found 293.1; [M+Na]⁺ calculated for 315.1, found 315.1. RP-HPLC Analysis, RT=11.7 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm). ¹H NMR (300 MHz, DMF) δ 7.8 (d, *J =* 8.5 Hz, 1H), 7.1 - 6.9 (m, 2H), 6.4 (s, 1H), 4.2 (t, *J* = 6.4 Hz, 2H), 3.6 (t, *J* = 6.3 Hz, 3H), 2.0 - 1.8 (m, 2H), 1.8 - 1.6 (m, 2H). D) ¹³C NMR (75 MHz, DMF) δ 171.2, 163.2, 161.0, 156.2, 151.0, 127.3, 113.7, 113.4, 113.3, 101.9, 69.3, 61.5, 38.0, 29.8, 26.3.

### Step 2:

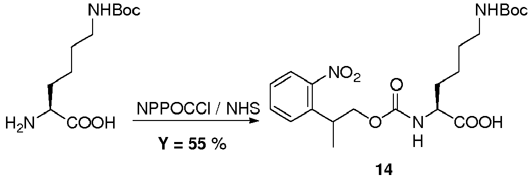

*N*ε-Boc-L-lysine Lys is modified in order to introduce the trigger moiety. 2-(Nitrophenyl)propyloxy carbonyl chloride NPPOCCl is activated by NHS at 0°C to form the reactive NPPOC-OSu intermediate which is added dropwise to the amino acid Lys and strirred at room temperature for 5h00. NPPOCLys(Boc) is isolated with 55 % yield after preparative RP-HPLC purification.
14: MALDI-TOF: matrix CHCA - Calculated for C₂₁H₃₁N₃O₈ [M+Na]⁺ 476.2, found 475.8; [M+K]⁺ calculated for 492.2, found 491.8. RP-HPLC Analysis, RT=21.1 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm). ESI spectrum calculated for C₁₂H₁₀O₅ [M+H]⁺ 454.2, found 454.4, [M-Boc]⁺ calculated for 354.2, found 354.3.

### Step 3' a:

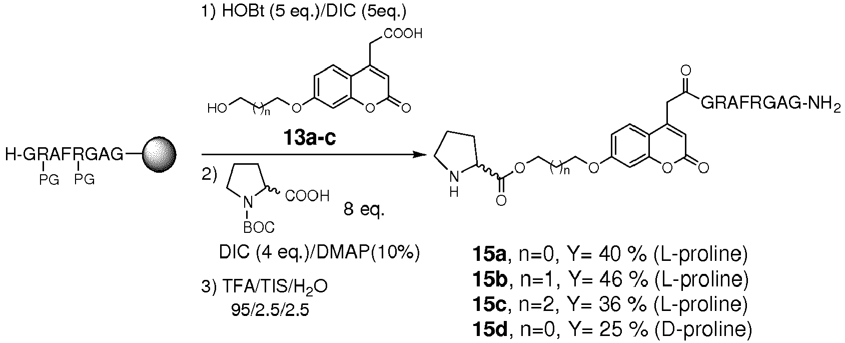

Proline esters 15a-d were synthesized in 3 steps starting from octapeptide resin Novasyn TGR. Protected octapeptide resin GRAFRGAG obtained from classic solid phase peptide synthesis using Fmoc/*tert*-butyl strategy is first modified on *N*-terminal position by hydroxy alkyl acids 13a-c (5 equivalents) using HOBt (5 equivalents) and DIC (5 equivalents) as coupling agent. After several washes with DCM (5×1 min.) and DMF (5×1 min.), the free hydroxyl group on the coumarine resins reacts with symmetrical anhydride previously prepared by mixing either L-Proline or D-Proline (8 equivalents), DIC (4 equivalents) and Dimethylaminopyridine DMAP (10 %) for 4 hours at room temperature. Consecutively, Proline was re-coupled following the same procedure for 16h00 at room temperature. Peptidyl resins were treated by a TFA solution with TIS (triisopropylsilane) as scavenger and residual crude peptides were purified by preparative RP-HPLC. Proline esters **15a-d** were isolated with yields between 25 and 46 %.
**15a:** MALDI-TOF: matrix CHCA - Calculated for C₅₁H₇₂N₁₆O₁₄ [M+H]⁺ 1133.5, found 1133.6 ; [M+Na]⁺ calculated 1155.5, found 1155.5. RP-HPLC Analysis, RT=9.8 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm). ESI spectrum calculated for C₁₂H₁₀O₅ [M+H]⁺ calculated 1133.5, found 1134.1, [M+2H]⁺/2 calculated 567.2, found 567.6.
15b: MALDI-TOF : matrix CHCA - Calculated for C₅₂H₇₄N₁₆O₁₄ [M+H]⁺ 1147.6, found 1147.6. RP-HPLC Analysis, RT=10.4 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm). ESI spectrum calculated for [M+2H]⁺/2 calculated 574.3, found 574.7.
**15c:** MALDI-TOF: matrix CHCA - Calculated for C₅₃H₇₆N₁₆O₁₄ [M+H]⁺ 1161.6, found 1161.5. RP-HPLC Analysis, RT=11.0 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm). ESI spectrum calculated for [M+2H]⁺/2 calculated 581.3, found 581.7.
15d: MALDI-TOF: matrix CHCA - Calculated for C₅₁H₇₂N₁₆O₁₄ [M+H]⁺ 1133.5, found 1133.5. RP-HPLC Analysis, RT=9.9 min. Buffer A H₂O-0.05% TFA / Buffer B 0.05% TFA CH₃CN/water (4/1) column C18 Nucleosil 100 Å 5 µm (2.1x250 mm) with a linear gradient 0-100%B in 30 minutes (1.0 mL/min, detection from 210 to 400 nm). ESI spectrum calculated for [M+2H]⁺/2 calculated 567.2, found 567.6, [M+H]⁺ calculated 1133.5, found 1134.9.

### Step 3'b:

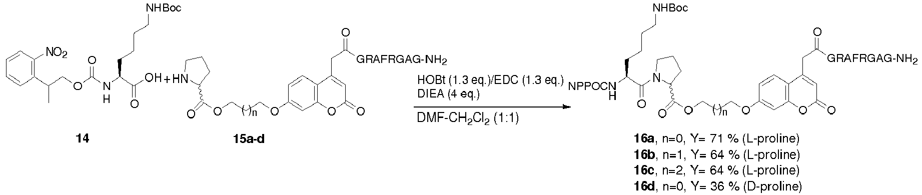

Coupling between NPPOCLys(Boc) **14** and Proline esters **16a-d** is carried out with *N*-(3-dimethylaminopropyl)-*N'*-ethyl carbodiimide EDC (1.3 equivalent) as coupling agent and HOBt (1.3 equivalent) as activator using DIEA (4 equivalents) as base in a 1:1 DMF-DCM mixture for 20 hours. Esters **16a-b** were obtained after purification with yields between 36 and 71 %.

16a, 16b, 16c and 16d are characterized by MALDI-TOF, RP-HPLC and ESI. Results are presented on figures 8 to 11.

### Step 3:

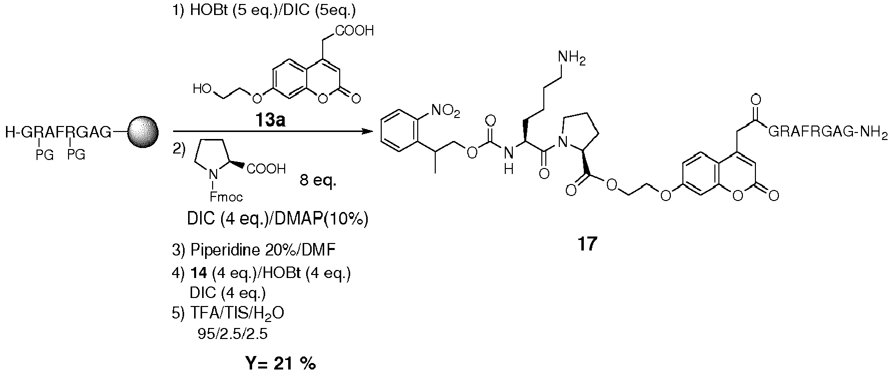

Preparation of amine 17 is carried out using the same solid phase strategy as described for 16a-d. Instead of Boc-Proline, Fmoc-L-Proline is introduced to the free hydroxyl groups on the coumarine resin. Double Fmoc deprotection with piperidine 20 % is realized on the resin.
After several washes with DCM (5×1 min.) and DMF (5×1 min.), NPPOCLys(Boc) (4 equivalents) is introduced on proline amine using DIC (4 equivalents) and HOBt (4 equivalents) in DMF at room temperature for 4h00. Final deprotection in TFA/TIS/H₂O yields amine 17 with 21% after preparative RP-HPLC purification.
17 is characterized by MALDI-TOF, RP-HPLC and ESI. Results are presented on figure 12.

### Step 4:

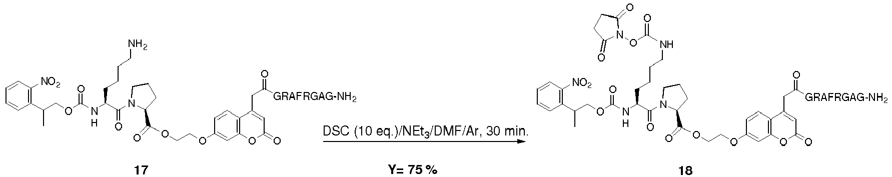

Amine ester **17** reacts as described above for compound 6 with DSC (10 equivalents) in DMF at room temperature. NHC **18** is isolated with 75 % yield after purification.
**18** is characterized by MALDI-TOF, RP-HPLC and ESI. Results are presented on figure 13.

### Example 6: Biomolecule Labeling according to the invention

Labeling of lyzozyme was endeavored with tag peptide NSC 18 as described for compound 8. MALDI-TOF and LC-MS analysis revealed around 70-80 % of lysozyme was labeled by one or several photocleavable tag peptide 18. The main product was lyzozyme labeled with label 19 (p=1) identified by a [M+H]⁺ mass of 15797 Da. Twice labeled lysozyme (p=2) was also characterized by a [M+H]⁺ mass of 17240 Da. Labeled lysozyme was purified by phosphate buffer three times washing under centrifugation (RCF=3000) at 8°C on a Vivaspin cartridge (cut-off 10000).

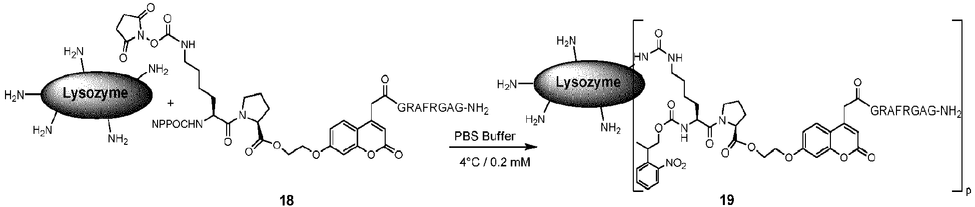

19 is characterized by MALDI-TOF, RP-HPLC and ESI. Results are presented on figure 14.

This procedure was also applied to NeutrAvidin labeling. NeutrAvidin is deglycosylated native avidin from egg whites. The protein has a more neutral isoelectric point pI = 6.3 and features less nonspecific binding properties. NeutrAvidin contains four identical subunits having a combined mass of 60,000 daltons, each subunit being constituted of 127 amino acids, and more particularly 10 primary amines including 9 Lysines. Labeling protocol was slightly modified by adding 10 to 20% of glycerol in order to avoid labeled NeutrAvidin **20** to precipitate. 16 µM NeutrAvidin solution (1 equivalent) in Phosphate Buffer Saline PBS and glycerol (10 %) at pH=7.4 was added to the photocleavable tag peptide NSC **18** (4 equivalents) at 4°C and solution was stirred at 4°C for 2 hours. Reaction medium RP-HPLC analysis revealed 80-90 % of NeutrAvidin has been labeled. Mass spectrometry analysis revealed 5 protein species containing starting unlabeled monomeric NeutrAvidin characterized by a [M+H]⁺ mass of 14840 Da. Main product was NeutrAvidin labeled with one label **20** (q=1) identified with a [M+H]=16291 Da. Twice and three and four times labeled proteins were also identified respectively with [M+H]⁺ mass of 17705 Da, 19147 Da and 20578 Da.
Labeled NeutrAvidin proteins were purified by dialysis cassette (cutoff 3,500 Da) in a PBS-glycerol (10%) solution at 4°C for 20 hours.

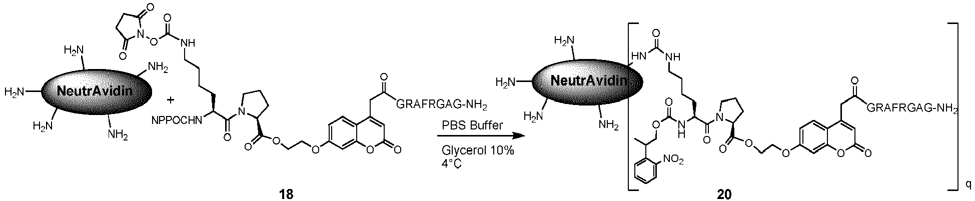

**20** is characterized by MALDI-TOF, RP-HPLC and ESI. Results are presented on figure 15.

### Example 7: Photocleavage of compounds 16a-d and release of tag 21a-c

Cleavage of photolabile NPPOC protecting group by irradiation of a 1 mM solution of tag peptide ester **16a-d** in phosphate buffer at room temperature for 15 minutes with a 100 Watt UV lamp (λ=365 nm) was assessed.

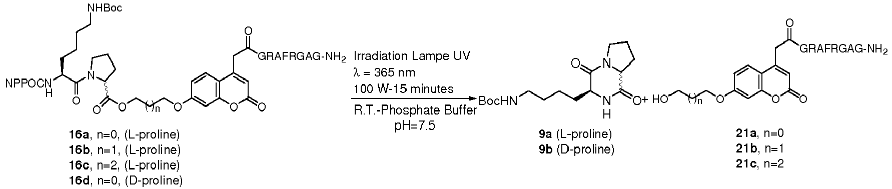

Irradiation of tag peptide esters **16a, 16b** and **16c** leads to emergence of diketopiperazine **9a** derived from L-proline and release of corresponding tag peptides **21a**, **21b** and 21c. Diketopiperazine **9a** was confirmed for a RT=12.3 min. with a [M+Na]⁺ mass of 348.3 Da by electrospray ionization ESI mass spectrometry analysis. Each tag peptide **21a-c** was confirmed by ESI and MALDI-TOF analysis respectively for RT=10.3, 11.0, 11.2 minutes with [M+H]⁺=1035.8, 1050.5, 1064.4 Da.
Regarding tag peptide ester **16d,** UV irradiation exposure leads to emergence of tag peptide **21a** and diketopiperazine **9b** issued from D-proline characterized by a RT=10.1 min. and [M+Na]⁺ mass of 348.3 Da by ESI mass spectrometry analysis.
**9a, 9b, 21a**, **21b** and **21c** are characterized by MALDI-TOF, RP-HPLC and ESI. Results are presented on figures 16 to 19.

Kinetic studies for UV irradiation exposure of tag peptide esters **5, 16a, 16b, 16c** and **16d** are summarized in figure 23 and allow comparing the rates of tag peptides **1a, 21a**, **21b** and **21c** releases. After 15 minutes UV exposure of a 1mM tag peptide ester in PBS solution, tag peptide release was detected and measured by a RP-HPLC analysis.
Tag peptide ester **5** displays a fast cyclization rate since 100 % of tag peptide **1a** was released in 15 minutes. Due to D-proline moiety, tag peptide ester **16d** presents fast rate release since 100 % tag peptide **21a** was detected after 4 hours. Slightly lower rate of tag peptide **21a** release was observed with 100% in 5 hours. Regarding tag peptide esters **16b** and **16c,** cyclization rates showed quite low since no more than 50 % of tag peptide **21b** and 21c were detected after 6 hours.
Figure 23 shows the tag release kinetics for UV irradiation exposure of tag peptide esters **5, 16a, 16b, 16c** and **16d.**

### Example 8: Photocleavage of compounds 19-20 and release of tag 21a

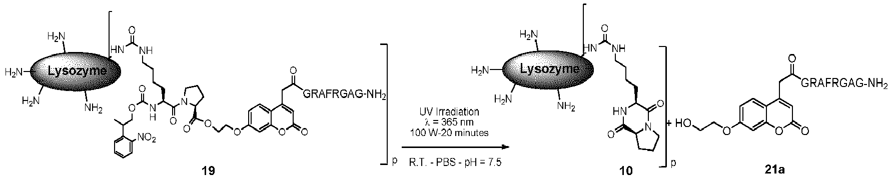

Irradiation at room temperature of tag peptide ester **19** leads to the release of tag peptide **21a** at a RT=10.3 min..Tag peptide **21a** was confirmed by MALDI-TOF mass spectrometry analysis with a [M+H]⁺=1035.8 Da. 10 and **21a** are characterized by MALDI-TOF, RP-HPLC and ESI. Results are presented on figure 20.

UV irradiation of tag peptide labeled NeutrAvidin **20** leads also to complete tag peptide **21a** release. Total conversion into diketopiperazine NeutrAvidin **22** from tag peptide **20** was confirmed by MALDI-TOF mass spectrometry analysis with a mass of [M+H]⁺=15015 Da and disapearance of multilabeled tag peptide NeutrAvidin **20.**

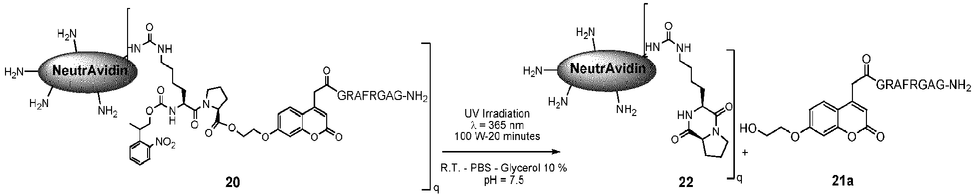

**22** and **21a** are characterized by MALDI-TOF, RP-HPLC and ESI. Results are presented on figure 21.

### Example 9: Preparation of different peptide tags

The peptides listed in table I below were synthesized as presented in example 1 to be used as tags in labels according to the invention.

| Peptide | Peptidic Sequence | Molecular Formula | m/z | Peptide D6 | m/z |
|---|---|---|---|---|---|
| **1** | GR**A**FRG**A**G-NH₂ | C35H57N15O9 | 831.45 | C35H51D6N15O9 | 837.48 |
| **2** | GR**A**FRG**S**G-NH₂ | C35H57N15O10 | 847.44 | C35H51D6N15O10 | 853.48 |
| **3** | GR**A**FRG**V**G-NH₂ | C37H61N15O9 | 859.48 | C37H55D6N15O9 | 865.51 |
| **4** | GR**A**FRG**N**G-NH₂ | C36H58N16O10 | 874.45 | C36H52D6N16O10 | 880.49 |
| **5** | GR**A**FRG**Q**G-NH₂ | C37H60N16O10 | 888.47 | C37H54D6N16O10 | 894.50 |
| 6 | GR**P**FRG**L**G-NH₂ | C40H65N15O9 | 899.51 | C40H59D6N15O9 | 905.55 |
| **7** | GR**N**FRG**N**G-NH₂ | C37H59N17O11 | 917.46 | C37H53D6N17O11 | 923.5 |
| **8** | GR**Q**FRG**N**G-NH₂ | C38H61N17O11 | 931.47 | C38H55D6N17O11 | 937.51 |
| **9** | GR**Q**FRG**Q**G-NH₂ | C39H63N17O11 | 945.49 | C39H57D6N17O11 | 951.53 |
| 10 | GR**Q**FRG**F**G-NH₂ | C43H64N16O10 | 964.5 | C43H58D6N16O10 | 970.54 |

The amino acids sequences of peptides 1 to 10 are respectively identified as SEQ ID No. 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10 and SEQ ID No. 11.

## Claims

1. Compound of the formula (I): 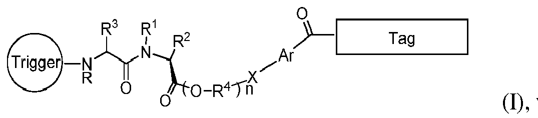 wherein:
- the tag is any group of atoms susceptible to be identified and/or detected by a technique of analysis,
- the trigger is a chemical group that is cleaved when subjected to a stimulus,
- n is 0 or 1,
- Ar is an aromatic group,
- R is selected in the group consisting of a hydrogen atom, a saturated or unsaturated, linear or branched, alkyl group, optionally interrupted by at least one heteroatom selected in the group consisting of oxygen O, nitrogen N, and sulfur S atoms, and optionally substituted with at least one group selected in the group consisting of a hydroxy group, an alkoxy group, an amino NH₂ group, a nitro NO₂ group, and a carboxylic acid COOH group,
- R¹ is a hydrogen atom and R² is selected in the group consisting of a hydrogen atom and a C1-C4 alkyl group, optionally substituted with a phenyl group; alternatively, R¹ and R² may form together and with the nitrogen and carbon atoms to which they are respectively linked a five membered ring,
- R³ is a chemical group of the formula -(CH₂)ₘ-CH₂-FG, wherein m is 0, 1, 2 or 3 and FG is a functional group that is able to react with a chemical group of a binding molecule.
- R⁴ is a C1-C4 alkyl group, and
- X is selected in the group consisting of oxygen O, NH and sulfur S.

2. Conjugate of the formula (II): 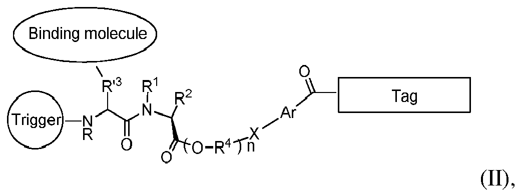 wherein
the tag, the trigger, R, R¹, R², R⁴, Ar, X and n are as defined in claim 1,
the binding molecule is a molecule able to link R³ as defined in claim 1, and R'³ is a chemical group of the formula -(CH₂)ₘ-CH₂-FG', wherein m is 0, 1, 2 or 3 and FG' is a chemical group obtained from the reaction of the FG group as defined in claim 1 and the binding molecule.

3. Conjugate according to claim 2, wherein the binding molecule is selected in the group consisting of antibodies, antigens, aptamers, proteins, peptides, oligosaccharides, polysaccharides, peptidoglycans, lipids, and nucleic acids such as DNA or RNA.

4. Compound according to claim 1 or conjugate according to claim 2 or 3, wherein the tag is a tag of known mass or a peptide, preferably a peptide of sequence SEQ ID No.1: Gly-Arg-Aa-Phe-Arg-Gly-Aa-Gly, wherein each Aa is selected independently in the group consisting of alanine, serine, valine, asparagine, glutamine, leucine and proline.

5. Compound or conjugate according to any of claims 1 to 4, wherein Ar is a 7-hydroxycoumarine derivative.

6. Method for preparing a conjugate according to any of claims 2 to 5, comprising contacting the binding molecule with at least one compound according to any of claims 1 to 6, preferably at a temperature about 4°C, and purifying the conjugate, preferably at a temperature about 4°C.

7. Method for detecting at least one target molecule in a sample, comprising the steps of:
(a) contacting the sample with at least one conjugate according to any of claims 2 to 5,
(b) removing any unbound conjugate from the sample,
(c) submitting the sample obtained after step (b) to the at least one appropriate stimulus to activate the conjugate(s), and
(d) detecting the release of the Tag-C(=O)-Ar-X-(R⁴-O)ₙ-H compounds by any appropriate analysis technique, preferably by MALDI mass spectrometry,
wherein the presence of the Tag-C(=O)-Ar-X-(R⁴-O)ₙ-H compounds indicates the presence of the corresponding target molecules in the sample,
wherein the Tag, Ar, X, R⁴, and n are as defined in any of claims 1 to 5.

8. Method of determining a risk of, or of detecting the predisposition to, or the presence of, a specific pathology in a subject, comprising detecting a target molecule specific of said pathology with at least a conjugate according to any of claims 2 to 5.

9. Method for determining a target molecule map in a tissue section, comprising the steps of:
(a) hybridizing said tissue section with at least one conjugate according to any of claims 2 to 5, suitable for binding the target molecule,
(b) spraying a matrix,
(c) scanning the tissue section surface and analyzing each adjacent spot with a MALDI mass spectrometer, wherein the MALDI laser is preferably used both to release the Tag-C(=O)-Ar-X-(R₄-O)ₙ-H compound and to induce the sample ionization, and wherein the resulting data of each spot is saved; and
(d) analyzing the obtained data in the molecular mass window(s) of each distinct Tag-C(=O)-Ar-X-(R₄-O)ₙ-H compound to create as many maps of the tissue section as the number of distinct studied target molecules.

10. Method for detecting at least one target molecule in a sample in an immuno-sensing assay, comprising the steps of:
(a) contacting the sample with a solid substrate having affixed thereto at least one compound able to link the at least one target molecule, wherein the contacting is performed under conditions which would permit the compound(s) to bind to the target molecule(s) if present in the sample,
(b) removing any unbound sample from the solid substrate,
(c) contacting the solid substrate obtained in step (a) with at least one conjugate according to any of claims 2 to 5, wherein the contacting is performed under conditions which would permit the conjugate(s) to bind to the target molecule(s) if present on the solid substrate,
(d) removing any unbound conjugate,
(e) submitting the sample obtained in step (c) to the appropriate stimulus (stimuli) to activate the conjugate(s), and
(f) detecting the release of the Tag-C(=O)-Ar-X-(R⁴-O)ₙ-H compound(s) by any appropriate analysis technique, preferably by MALDI mass spectrometry,
wherein the presence of the Tag-C(=O)-Ar-X-(R⁴-O)ₙ-H compound(s) indicates the presence of the corresponding target molecule(s) in the sample.

11. Method according to claim 10, wherein, for each target molecule, there is:
- at least one compound able to link the target molecule affixed to the solid substrate of step (a), and
- at least one conjugate according to any of claims 2 to 5 which binds to the target molecule concurrently with the compound able to link the target molecule, and
the tag of the at least one conjugate has a different signal of detection, in particular a different mass, than that of the tag of the conjugates that bind any other target molecule.

12. Method for detecting at least one target molecule in a sample in an immuno-sensing assay, comprising the steps of:
(a) contacting the sample with a solid substrate which binds to the target molecule(s), wherein the contacting is performed under conditions which would permit the support to bind to the target molecule(s) if present in the sample,
(b) removing any unbound sample from the solid substrate,
(c) contacting the solid substrate obtained in step (a) with at least one conjugate according to any of claims 2 to 5 that binds to the target molecule(s) concurrently with the support, wherein the contacting is performed under conditions which would permit the conjugate(s) to bind to the target molecule(s) if present on the solid substrate,
(d) removing any unbound conjugate,
(e) submitting the sample obtained in step (d) to the appropriate stimulus (stimuli) to activate the conjugate(s), and
(f) detecting the release of the Tag-C(=O)-Ar-X-(R⁴-O)ₙ-H compound(s) by any appropriate analysis technique, preferably by MALDI mass spectrometry,
wherein the presence of the Tag-C(=O)-Ar-X-(R⁴-O)ₙ-H compound(s) indicates the presence of the corresponding target molecule(s) in the sample.

13. Method according to claim 12, wherein the solid substrate of step (a) binds to several target molecules, and, for each target molecule, there is at least one conjugate according to any of claims 2 to 5 which binds to the target molecule concurrently with the solid substrate, and the tag of the at least one conjugate has a different signal of detection, in particular a different mass, than that of the tag of the conjugates that bind any other target molecule.

14. Composition of matter comprising:
(a) a solid substrate,
(b) a compound bound to the solid substrate, wherein the compound is able to link at least one target molecule,
(c) the target molecule linked to the compound, and
(d) a conjugate according to any of claims 2 to 5 which binds to the target molecule concurrently to the compound, wherein the conjugate is bound to the target molecule.

15. Kit for detecting a target molecule in a sample comprising:
(a) a solid substrate,
(b) a compound able to link a target molecule for affixing to the solid substrate,
(c) a conjugate according to any of claims 2 to 5 that links the target molecule concurrently with the compound, and
(d) instructions for using the kit to detect the target molecule in the sample.

16. Kit for detecting a target molecule in a sample comprising:
(a) a solid substrate affixed with a compound able to link the target molecule, and
(b) a conjugate according to any of claims 2 to 5, which conjugate binds the target molecule, and
(c) instructions for using the kit to detect the target molecule in the sample.

17. Kit for detecting a target molecule in a sample comprising:
(a) a solid substrate which binds the target molecule,
(b) a conjugate according to any of claims 2 to 5, which conjugate binds the target molecule, and
(c) instructions for using the kit to detect the target molecule in the sample.

18. Kit for detecting at least one target molecule in a sample comprising:
(a) a solid substrate,
(b) a plurality of compounds able to link target molecules for affixing to the solid substrate, wherein for each target molecule there is at least one compound able to link said target molecule,
(c) a plurality of conjugates according to any of claims 2 to 5, wherein for each target molecule there is at least one conjugate that links said target molecule concurrently with the compound, and wherein the tag of each conjugate has a different signal of detection, in particular a different mass, than that of the tag of the conjugates that bind any other target molecule, and
(d) instructions for using the kit to detect at least one target molecule in the sample.

19. Kit for detecting at least one target molecule in a sample comprising:
(a) a solid substrate having affixed thereto a plurality of compounds able to link target molecules, wherein for each target molecule there is at least one compound able to link said target molecule,
(b) a plurality of conjugates according to any of claims 2 to 5, wherein for each target molecule there is at least one conjugate that links said target molecule concurrently with the compound, and wherein the tag of each conjugate has a different signal of detection, in particular a different mass, than that of the tag of the conjugates that bind any other target molecule, and
(c) instructions for using the kit to detect at least one target molecule in the sample.

20. Kit for detecting at least one target molecule in a sample comprising:
(a) a solid substrate which binds each of the target molecules,
(b) a plurality of conjugates according to any of claims 2 to 5, wherein for each target molecule there is at least one conjugate that links said target molecule and wherein the tag of each conjugate has a different signal of detection, in particular a different mass, than that of the tag of the conjugates that bind any other target molecule, and
(c) instructions for using the kit to detect at least one target molecule in the sample.
